# EUROPÄISCHE PATENTSCHRIFT

(11) **EP 2 831 084 B1**
(45) Veröffentlichungstag und Bekanntmachung des Hinweises auf die Patenterteilung: **23.03.2016**
(21) Anmeldenummer: 13711906.1
(22) Anmeldetag: 25.03.2013
(51) Int. Cl.: C07D 513/04, A01N 43/90

(54) **HERBIZID UND INSEKTIZID WIRKSAME THIAZOLOPYRIDINONE**
HERBICIDAL AND INSECTICIDAL THIAZOLOPYRIDINONES
THIAZOLOPYRIDINONE À EFFET HERBICIDE ET INSECTICIDE

(30) Priorität: 27.03.2012 EP 12161413
(43) Veröffentlichungstag der Anmeldung: 04.02.2015
(73) Patentinhaber: Bayer Intellectual Property GmbH, 40789 Monheim (DE)
(72) Erfinder: WALDRAFF, Christian, 61118 Bad Vilbel (DE); LEHR, Stefan, 65835 Liederbach (DE); ANGERMANN, Alfred, 65830 Kriftel (DE); DIETRICH, Hansjörg, 65835 Liederbach am Taunus (DE); DITTGEN, Jan, 60316 Frankfurt (DE); GATZWEILER, Elmar, 61231 Bad Nauheim (DE); ROSINGER, Christopher, Hugh, 65719 Hofheim (DE); BECKER, Angela, 40235 Düsseldorf (DE)
(74) Vertreter: BIP Patents
(86) Internationale Anmeldenummer: PCT/EP2013/056311
(87) Internationale Veröffentlichungsnummer: WO 2013/144096

(56) Entgegenhaltungen:
- WO-A1-2011/051212
- WO-A1-2012/028582

## Beschreibung

Die Erfindung betrifft das technische Gebiet der Herbizide und der Insektizide, insbesondere das der Herbizide zur selektiven Bekämpfung von Unkräutern und Ungräsern sowie der Bekämpfung von Insekten in Nutzpflanzenkulturen.

Speziell betrifft sie substituierte Pyridinon-Derivate, Verfahren zu ihrer Herstellung und ihre Verwendung als Herbizide.

Verschiedene Schriften beschreiben herbizid wirksame Pyridinone, die mit fünf- oder sechsgliedrigen heterocyclischen Ringen ein kondensiertes Ringsystem bilden. WO2011/051212 offenbart Pyridinone, die mit ausgewählten fünfgliedrigen Heterocyclen kondensiert sind und die in 3-Position des Pyridinrings durch Aryl- und Heteroaryl-Reste substituiert sind. Die WO 2012/028582 A1 offenbart Pyridinone, die mit ausgewählten fünf- und sechsgliedrigen Heterocyclen kondensiert sind und die in 3-Position des Pyridinrings durch Aryl-Reste substituiert sind.

Die aus diesen Schriften bekannten Verbindungen zeigen jedoch häufig eine nicht ausreichende herbizide Wirksamkeit. Aufgabe der vorliegenden Erfindung ist daher die Bereitstellung weiterer herbizid wirksamer Verbindungen.

Es wurde gefunden, dass Thiazolopyridinone, die in 3-Position des Pyridinrings durch spezielle aromatische Reste substituiert sind, als Herbizide besonders gut geeignet sind. Ein Gegenstand der vorliegenden Erfindung sind somit Diketopyridine der Formel (I) oder deren Salze 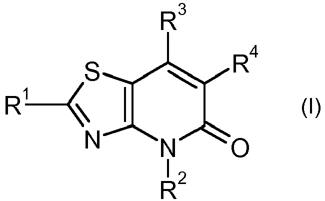 worin
R¹ bedeutet Wasserstoff, Halogen, Nitro, Cyano,
   jeweils durch n Halogenatome substituiertes (C₁-C₆)-Alkyl, (C₂-C₆)-Alkenyl, (C₂-C₆)-Alkinyl, (C₁-C₆)-Alkoxy, (C₁-C₆)-Alkylthio, (C₁-C₆)-Alkylsulfinyl, (C₁-C₆)-Alkylsulfonyl, (C₃-C₆)-Cycloalkyl, (C₁-C₆)-Alkoxy-(C₁-C₆)-alkyl, Di-(C₁-C₆)-alkoxy-(C₁-C₆)-alkyl oder (C₁-C₆)-Alkylthio-(C₁-C₆)-alkyl,
R² bedeutet Wasserstoff,
   jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₆)-Alkoxy-(C₁-C₆)-alkyl oder Di-(C₁-C₆)-alkoxy-(C₁-C₆)-alkyl, (C₁-C₆)-Al kylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, (C₃-C₆)-Cycloalkyl-(C₁-C₆)-alkyl, Cyano-(C₁-C₆)-alkyl, oder jeweils durch n Reste aus der Gruppe bestehend aus Halogen, (C₁-C₆)-Alkyl und (C₁-C₆)-Alkoxy substituiertes Phenyl oder Benzyl,
R³ bedeutet Hydroxy, O-C(=O)R⁷, O-C(=L)MR⁸, O-SO2R⁹, O-P(=L)R¹⁰R¹¹, O-C(=L)NR¹²R¹³, O-E oder O-R¹⁴,
R⁴ bedeutet R^{4a},
R^{4a} bedeutet durch n Reste R⁵ und einen Rest R⁶ substituiertes Aryl,
R⁵ bedeutet Wasserstoff, Halogen, Nitro, Cyano,
   jeweils durch n Halogenatome substituiertes (C₁-C₆)-Alkyl, (C₂-C₆)-Alkenyl, (C₂-C₆)-Alkinyl, (C₁-C₆)-Alkoxy, (C₁-C₆)-Alkylthio, (C₁-C₆)-Alkylsulfinyl, (C₁-C₆)-Alkylsulfonyl, (C₃-C₆)-Cycloalkyl, (C₁-C₆)-Alkoxy-(C₁-C₆)-alkyl oder (C₁-C₆)-Alkylthio-(C₁-C₆)-alkyl,
R⁶ bedeutet jeweils durch s Reste R⁵ substituiertes Aryl oder Heteroaryl,
E bedeutet ein Metallionäquivalent oder ein Ammoniumion,
L bedeutet Sauerstoff oder Schwefel,
M bedeutet Sauerstoff oder Schwefel,
R⁷ bedeutet jeweils durch n Halogenatome substituiertes (C₁-C₆)-Alkyl, (C₂-C₆)-Alkenyl, (C₁-C₆)-Alkoxy-(C₁-C₆)-alkyl, Di-(C₁-C₆)-alkoxy-(C₁-C₆)-alkyl oder (C₁-C₆)-Alkylthio-(C₁-C₆)-alkyl,
   einen durch n Reste aus der Gruppe bestehend aus Halogen, (C₁-C₆)-Alkyl und (C₁-C₆)-Alkoxy substituierten, vollständig gesättigten, 3- bis 6-gliedrigen Ring bestehend aus 3 bis 5 Kohlenstoffatomen und 1 bis 3 Heteroatomen aus der Gruppe bestehend aus Sauerstoff, Schwefel und Stickstoff,
   oder jeweils durch n Reste aus der Gruppe bestehend aus Halogen, (C₁-C₆)-Alkyl und (C₁-C₆)-Alkoxy substituiertes (C₃-C₆)-Cycloalkyl, Phenyl, Phenyl-(C₁-C₆)-alkyl, Phenoxy-(C₁-C₆)-alkyl oder Heteroaryloxy-(C₁-C₆)-alkyl,
R⁸ bedeutet jeweils durch n Halogenatome substituiertes (C₁-C₆)-Alkyl, (C₂-C₆)-Alkenyl, (C₁-C₆)-Alkoxy-(C₁-C₆)-alkyl oder Di-(C₁-C₆)-alkoxy-(C₁-C₆)-alkyl, oder jeweils durch n Reste aus der Gruppe bestehend aus Halogen, (C₁-C₆)-Alkyl und (C₁-C₆)-Alkoxy substituiertes (C₃-C₆)-Cycloalkyl, Phenyl oder Benzyl,
R⁹, R¹⁰, R¹¹ bedeuten unabhängig voneinander jeweils durch n Halogenatome substituiertes (C₁-C₆)-Alkyl, (C₁-C₆)-Alkoxy, N-(C₁-C₆)-Alkylamino, N,N-Di-(C₁-C₆)-Alkylamino, (C₁-C₆)-Alkylthio, (C₂-C₆)-Alkenyl oder (C₃-C₆)-Cycloalkylthio, oder jeweils durch n Reste aus der Gruppe bestehend aus Halogen, (C₁-C₆)-Alkyl und (C₁-C₆)-Alkoxy substituiertes Phenyl, Benzyl, Phenoxy oder Phenylthio,
R¹² , R¹³ bedeuten unabhängig voneinander jeweils Wasserstoff, durch n Halogenatome substituiertes (C₁-C₆)-Alkyl, (C₃-C₆)-Cycloalkyl, (C₂-C₆)-Alkenyl, (C₁-C₆)-Alkoxy oder (C₁-C₆)-Alkoxy-(C₁-C₆)-alkyl, jeweils durch n Reste aus der Gruppe bestehend aus Halogen, (C₁-C₆)-Alkyl und (C₁-C₆)-Alkoxy substituiertes Phenyl oder Benzyl, oder R¹² und R¹³ bilden gemeinsam mit dem N-Atom, an das sie gebunden sind, einen 3- bis 6-gliedrigen Ring enthaltend 2 bis 5 Kohlenstoffatomen und jeweils 0 oder 1 Sauerstoff- oder Schwefelatome,
R¹⁴ bedeutet jeweils durch n Halogenatome substituiertes (C₁-C₆)-Alkyl, (C₂-C₆)-Alkenyl, (C₂-C₆)-Alkinyl, (C₁-C₆)-Alkoxy-(C₁-C₆)-alkyl, (C₁-C₆)-Alkylthio-(C₁-C₆)-alkyl oder Di-(C₁-C₆)-alkoxy-(C₁-C₆)-alkyl,
   durch n Reste aus der Gruppe bestehend aus Halogen, (C₁-C₆)-Alkyl und (C₁-C₆)-Alkoxy substituiertes (C₃-C₆)-Cycloalkyl,
   einen durch n Reste aus der Gruppe bestehend aus Halogen, (C₁-C₆)-Alkyl und (C₁-C₆)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
   durch n Reste aus der Gruppe bestehend aus Halogen, (C₁-C₆)-Alkyl und (C₁-C₆)-Alkoxy substituiertes Phenyl, Phenyl-(C₁-C₆)-alkyl, Heteroaryl, Phenoxy-(C₁-C₆)-alkyl oder Heteroaryloxy-(C₁-C₆)-alkyl,
n bedeutet 0, 1, 2 oder 3,
s bedeutet 0, 1, 2 , 3 oder 4.

Alkyl bedeutet gesättigte, geradkettige oder verzweigte Kohlenwasserstoffreste mit 1 bis 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methyl-propyl.

Halogenalkyl bedeutet geradkettige oder verzweigte Alkylgruppen mit 1 bis 8 Kohlenstoffatomen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Alkenyl bedeutet ungesättigte, geradkettige oder verzweigte Kohlenwasserstoffreste mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Alkinyl bedeutet geradkettige oder verzweigte Kohlenwasserstoffreste mit 2 bis 8 Kohlenstoffatomen und einer Dreifachbindung in einer beliebigen Position, z.B. C₂-C₆-Alkinyl wie Ethinyl, 1-Propinyl, 2-Propinyl (oder Propargyl), 1-Butinyl, 2-Butinyl, 3-Butinyl, 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Alkoxy bedeutet gesättigte, geradkettige oder verzweigte Alkoxyreste mit 1 bis 8 Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy, Halogenalkoxy bedeutet geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy.

Alkylthio bedeutet gesättigte, geradkettige oder verzweigte Alkylthioreste mit 1 bis 8 Kohlenstoffatomen, z.B. C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methyl-pentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio.
Halogenalkylthio bedeutet geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio.

Aryl bedeutet Phenyl oder Naphthyl.

Heteroaryl bedeutet insbesondere 2-Furyl, 3-Furyl, 2-Thienyl, 3-Thienyl, 1-Pyrrolyl, 2-Pyrrolyl, 3-Pyrrolyl, 3-lsoxazolyl, 4-lsoxazolyl, 5-lsoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-1-yl, 1,2,4-Triazol-3-yl, 1,2,4-Triazol-4-yl, 1,2,4-Triazol-5-yl, 1,2,3-Triazol-1-yl, 1,2,3-Triazol-2-yl, 1,2,3-Triazol-4-yl, Tetrazol-1-yl, Tetrazol-2-yl, Tetrazol-5-yl, Indol-1-yl, Indol-2-yl, Indol-3-yl, Isoindol-1-yl, Isoindol-2-yl, Benzofur-2-yl, Benzothiophen-2-yl, Benzofur-3-yl, Benzothiophen-3-yl, Benzoxazol-2-yl, Benzothiazol-2-yl, Benzimidazol-2-yl, Indazol-1-yl, Indazol-2-yl, Indazol-3-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl oder 1,2,4-Triazin-6-yl. Dieses Heteroaryl ist - sofern nicht anders angegeben -jeweils unsubstituiert oder jeweils einfach oder mehrfach gleich oder verschieden substituiert durch Reste ausgewählt aus Fluor, Chlor, Brom, lod, Cyano, Hydroxy, Mercapto, Amino, Methyl, Ethyl, n-Propyl, Isopropyl, n-Butyl, Isobutyl, sek-Butyl, tert-Butyl, Cyclopropyl, 1-Chlorcyclopropyl, Vinyl, Ethinyl, Methoxy, Ethoxy, Isopropoxy, Methylthio, Ethylthio, Trifluormethylthio, Chlordifluormethyl, Dichlorfluormethyl, Chlorfluormethyl, Chlormethyl, Dichlormethyl, Trichlormethyl, Fluormethyl, Difluormethyl, Trifluormethyl, 2,2,2-Trifluorethyl, Trifluormethoxy, Trifluormethylthio, 2,2,2-Trifluor-thoxy, 2,2-Dichlor-2-fluorethyl, 2,2-Difluor-2-chlorethyl, 2-Chlor-2-fluorethyl, 2,2,2-Trichlorethyl, 2,2,2-Trifluorethyl, 2-Fluorethyl, 2,2-Difluorethyl, 2-Methoxyethoxy, Acetyl, Propionyl, Methoxycarbonyl, Ethoxycarbonyl, N-Methylamino, N,N-Dimethylamino, N-Ethylamino, N,N-Diethylamino, Aminocarbonyl, Methylaminocarbonyl, Dimethylaminocarbonyl, Dimethylcarbamoylamino, Methoxycarbonylamino, Methoxycarbonyloxy, Ethoxycarbonylamino, Ethoxycarbonyloxy, Methylsulfamoyl, Dimethylsulfamoyl, Phenyl oder Phenoxy.

Unter einem gesättigten oder ungesättigten fünfgliedrigen Heterocyclus ist ein fünfgliedriges Ringsystem zu verstehen, das außer Kohlenstoffatomen 1 bis 4 Heteroatome aus der Gruppe Sauerstoff, Schwefel und Stickstoff enthält. Beispiele für einen solchen Heterocyclus sind Furan, Thiophen, 1,2-Oxazol, 1,3-Oxazol, 1,2-Thiazol, 1,3-Thiaxazol, Imidazol, Pyrazol, 1,2-Diazol, 1,2,5-Oxadiazol und jeweils deren ungesättigten und teilweise gesättigten Analoga.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im Folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Ein Metallionäquivalent bedeutet ein Metallion mit einer positiven Ladung wie Na⁺, K⁺, (Mg²⁺)_{1/2}, (Ca²⁺)_{1/2}, MgH⁺, CaH⁺, (Al³⁺)_{1/3} (Fe²⁺)_{1/2} oder (Fe³⁺)_{1/3}.

Halogen bedeutet Fluor, Chlor, Brom und Jod.

Ist eine Gruppe mehrfach durch Reste substituiert, so ist darunter zu verstehen, daß diese Gruppe durch ein oder mehrere gleiche oder verschiedene der genannten Reste substituiert ist.

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel (I) Amino, Alkylamino oder andere, basische Eigenschaften induzierende Gruppen, so können diese Verbindungen mit Säuren zu Salzen umgesetzt werden oder fallen durch die Synthese direkt als Salze an.

Beispiele für anorganische Säuren sind Halogenwasserstoffsäuren wie Fluorwasserstoff, Chlorwasserstoff, Bromwasserstoff und lodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oderdiphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure. Als Metallionen kommen insbesondere die Ionen der Elemente der zweiten Hauptgruppe, insbesondere Calcium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄)-Alkyl-Gruppen, Mono-, Di- und Trialkanolamine von (C₁-C₄)-Alkanolen, Cholin sowie Chlorcholin.

Die Verbindungen der Formel (I) können je nach Art und Verknüpfung der Substituenten als Stereoisomere vorliegen. Sind beispielsweise ein oder mehrere asymmetrisch substiuierte Kohlenstoffatome oder Sulfoxide vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der Formel (I) umfaßt, jedoch nicht spezifisch definiert sind.

In allen nachfolgend genannten Formeln haben die Substituenten und Symbole, sofern nicht anders definiert, dieselbe Bedeutung wie unter Formel (I) beschrieben.

Bevorzugt sind Thiazolopyridinone der Formel (I), worin
R¹ bedeutet Wasserstoff,
   jeweils durch n Halogenatome substituiertes (C₁-C₆)-Alkyl, (C₁-C₆)-Alkoxy, (C₁-C₆)-Alkylthio, (C₁-C₆)-Alkylsulfonyl, (C₃-C₆)-Cycloalkyl, (C₁-C₆)-Alkoxy-(C₁-C₆)-alkyl oder (C₁-C₆)-Alkylthio-(C₁-C₆)-alkyl,
R² bedeutet Wasserstoff,
   jeweils durch n Halogenatome substituiertes (C₁-C₆)-Alkyl, (C₂-C₆)-Alkenyl, (C₃-C₆)-Alkinyl, (C₁-C₆)-Alkoxy-(C₁-C₆)-alkyl, (C₁-C₆)-Alkylthio-(C₁-C₆)-alkyl, (C₃-C₆)-Cycloalkyl-(C₁-C₆)-alkyl, Cyano-(C₁-C₆)-alkyl,
   oder jeweils durch n Reste aus der Gruppe bestehend aus Halogen, (C₁-C₆)-Alkyl und (C₁-C₆)-Alkoxy substituiertes Phenyl oder Benzyl,
R³ bedeutet Hydroxy, O-C(=O)R⁷, O-C(=L)MR⁸, O-SO₂R⁹, O-P(=L)R¹⁰R¹¹, O-C(=L)NR¹²R¹³, O-E oder O-R¹⁴,
R⁴ bedeutet R^{4a},
R^{4a} bedeutet durch einen, zwei oder drei Reste R⁵ und einen Rest R⁶ substituiertes Phenyl,
R⁵ bedeutet Wasserstoff, Halogen,
   jeweils durch n Halogenatome substituiertes (C₁-C₆)-Alkyl, (C₂-C₆)-Alkenyl, (C₂-C₆)-Alkinyl, (C₁-C₆)-Alkoxy, (C₃-C₆)-Cycloalkyl oder (C₁-C₆)-Alkoxy-(C₁-C₆)-alkyl,
R⁶ bedeutet durch n Reste R⁵ substituiertes Phenyl oder durch einen, zwei oder drei Reste R⁵ substituiertes Heteroaryl,
E bedeutet ein Metallionäquivalent oder ein Ammoniumion,
L bedeutet Sauerstoff oder Schwefel,
M bedeutet Sauerstoff oder Schwefel,
R⁷ bedeutet jeweils durch n Halogenatome substituiertes (C₁-C₆)-Alkyl, (C₂-C₆)-Alkenyl, (C₁-C₆)-Alkoxy-(C₁-C₆)-alkyl, Di-(C₁-C₆)-alkoxy-( C₁-C₆)-alkyl oder (C₁-C₆)-Alkylthio-(C₁-C₆)-alkyl,
   einen durch n Reste aus der Gruppe bestehend aus Halogen, (C₁-C₆)-Alkyl und (C₁-C₆)-Alkoxy substituierten, vollständig gesättigten, 3- bis 6-gliedrigen Ring bestehend aus 3 bis 5 Kohlenstoffatomen und 1 bis 3 Heteroatomen aus der Gruppe bestehend aus Sauerstoff, Schwefel und Stickstoff,
   oder jeweils durch n Reste aus der Gruppe bestehend aus Halogen, (C₁-C₆)-Alkyl und (C₁-C₆)-Alkoxy substituiertes (C₃-C₆)-Cycloalkyl, Phenyl, Phenyl-(C₁-C₆)-alkyl, Phenoxy-(C₁-C₆)-alkyl oder Heteroaryloxy-(C₁-C₆)-alkyl,
R⁸ bedeutet jeweils durch n Halogenatome substituiertes (C₁-C₆)-Alkyl, (C₂-C₆)-Alkenyl, (C₁-C₆)-Alkoxy-(C₁-C₆)-alkyl oder Di-(C₁-C₆)-alkoxy-(C₁-C₆)-alkyl, oder jeweils durch n Reste aus der Gruppe bestehend aus Halogen, (C₁-C₆)-Alkyl und (C₁-C₆)-Alkoxy substituiertes (C₃-C₆)-Cycloalkyl, Phenyl oder Benzyl,
R⁹, R¹⁰, R¹¹ bedeuten unabhängig voneinander jeweils durch n Halogenatome substituiertes (C₁-C₆)-Alkyl, (C₁-C₆)-Alkoxy, N-(C₁-C₆)-Alkylamino, N,N-Di-(C₁-C₆)-Alkylamino, (C₁-C₆)-Alkylthio, (C₂-C₆)-Alkenyl oder (C₃-C₆)-Cycloalkylthio, oder jeweils durch n Reste aus der Gruppe bestehend aus Halogen, (C₁-C₆)-Alkyl und (C₁-C₆)-Alkoxy substituiertes Phenyl, Benzyl, Phenoxy oder Phenylthio,
R¹², R¹³ bedeuten unabhängig voneinander jeweils Wasserstoff,
   durch n Halogenatome substituiertes (C₁-C₆)-Alkyl, (C₃-C₆)-Cycloalkyl, (C₂-C₆)-Alkenyl, (C₁-C₆)-Alkoxy oder (C₁-C₆)-Alkoxy-(C₁-C₆)-alkyl,
   jeweils durch n Reste aus der Gruppe bestehend aus Halogen, (C₁-C₆)-Alkyl und (C₁-C₆)-Alkoxy substituiertes Phenyl oder Benzyl,
   oder R¹² und R¹³ bilden gemeinsam mit dem N-Atom, an das sie gebunden sind, einen 3- bis 6-gliedrigen Ring enthaltend 2 bis 5 Kohlenstoffatomen und jeweils 0 oder 1 Sauerstoff- oder Schwefelatome,
R¹⁴ bedeutet jeweils durch n Halogenatome substituiertes (C₁-C₆)-Alkyl, (C₂-C₆)-Alkenyl, (C₂-C₆)-Alkinyl, (C₁-C₆)-Alkoxy-(C₁-C₆)-alkyl, (C₁-C₆)-Alkylthio-(C₁-C₆)-alkyl oder Di-(C₁-C₆)-alkoxy-(C₁-C₆)-alkyl,
   durch n Reste aus der Gruppe bestehend aus Halogen, (C₁-C₆)-Alkyl und (C₁-C₆)-Alkoxy substituiertes (C₃-C₆)-Cycloalkyl,
   einen durch n Reste aus der Gruppe bestehend aus Halogen, (C₁-C₆)-Alkyl und (C₁-C₆)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
   durch n Reste aus der Gruppe bestehend aus Halogen, (C₁-C₆)-Alkyl und (C₁-C₆)-Alkoxy substituiertes Phenyl, Phenyl-(C₁-C₆)-alkyl, Heteroaryl, Phenoxy-(C₁-C₆)-alkyl oder Heteroaryloxy-(C₁-C₆)-alkyl,
n bedeutet 0, 1, 2 oder 3,
s bedeutet 0, 1, 2 , 3 oder 4.

Besonders bevorzugt sind die in den nachfolgenden angegebenen Verbindungen der Formel (I).

**Tabelle 1: Erfindungsgemäße Verbindungen der Formel (I), worin R¹ Wasserstoff, R² 2,2-Difluorethyl und R³ Hydroxy bedeutet (die Verbindungen 39, und 50 und 51 sind Vergleichsverbindungen).**

| 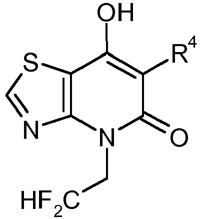 | |
|---|---|
| Nr. | R⁴ |
| 1 | 2-Chlor-4-(5-chlor-2-thienyl)-6-ethylphenyl |
| 2 | 2-Chlor-4-(2,4-dichlorophenyl)-6-ethylphenyl |
| 3 | 2-Chlor-4-(4-chlorophenyl)-6-ethylphenyl |
| 4 | 2,6-Dichlor-3-(5-chlor-2-thienyl)phenyl |
| 5 | 2,6-Dichlor-3-(2,4-dichlorphenyl)phenyl |
| 6 | 3-(4-Chlorphenyl)-2,6-dichlorphenyl |
| 7 | 2-Chlor-3-(5-chlor-2-thienyl)-6-fluorphenyl |
| 8 | 2-Chlor-3-(2,4-dichlorphenyl)-6-fluorphenyl |
| 9 | 2-Chlor-3-(4-chlorphenyl)-6-fluorphenyl |
| 10 | 2-Chlor-4-(4-chlorphenyl)phenyl |
| 11 | 2-Chlor-3-(4-chlorphenyl)phenyl |
| 12 | 5-(4-Chlorphenyl)-2-methylphenyl |
| 13 | 3-(3-Chlorphenyl)-2,6-dichlorphenyl |
| 14 | 2,6-Dichlor-(3-phenyl)phenyl |
| 15 | 2-Chlor-4-(4-fluorphenyl)phenyl |
| 16 | 4-(4-Bromphenyl)-2-chlorphenyl |
| 17 | 2-Chlor-4-(4-iodphenyl)phenyl |
| 18 | 2-Brom-4-(4-chlorphenyl)phenyl |
| 19 | 2-Brom-4-(4-fluorphenyl)phenyl |
| 20 | 2-Brom-4-(4-iodphenyl)phenyl |
| 21 | 4-(4-Chlorphenyl)-3-fluorphenyl |
| 22 | 4-(4-Bromphenyl)-3-fluorphenyl |
| 23 | 3-Fluor-4-(4-iodphenyl)phenyl |
| 24 | 3-Fluor-4-(4-fluorphenyl)phenyl |
| 25 | 4-(4-Chlorphenyl)-2-iodphenyl |
| 26 | 4-(4-Bromphenyl)-2-iodphenyl |
| 27 | 4-(4-Fluorphenyl)-2-iodphenyl |
| 28 | 2-lod-4-(4-iodphenyl)phenyl |
| 29 | 4-(4-Fluorphenyl)-2-trifluormethylphenyl |

| Nr. | R⁴ |
|---|---|
| 30 | 4-(4-Chlorphenyl)-2-trifluormethylphenyl |
| 31 | 4-(4-lodphenyl)-2-trifluormethylphenyl |
| 32 | 4-(4-Bromphenyl)-2-trifluormethylphenyl |
| 39 | 2-Chlor-3-thiophenyl |
| 50 | 5-Chlor-2-thiophenyl |
| 51 | 2-Chlor-1,3-thiazol-5-yl |
| 114 | 3-(4,5-Dihydro-1,2-oxazol-3-yl)-2-(trifluormethyl)phenyl |

Tabelle 2: Erfindungsgemäße Verbindungen der Formel (I), worin R¹ Wasserstoff, R² 2,2,2-Trifluorethoxy und R³ Hydroxy bedeutet und R⁴ die in Tabelle 1 angegebenen Bedeutungen hat:

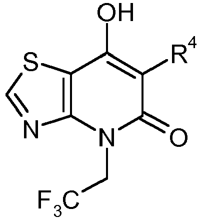

Tabelle 3: Erfindungsgemäße Verbindungen der Formel (I), worin R¹ Wasserstoff, R² Methyl und R³ Hydroxy bedeutet und R⁴ die in Tabelle 1 angegebenen Bedeutungen hat:

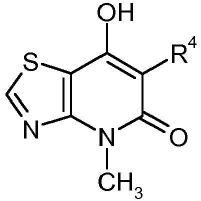

Tabelle 4: Erfindungsgemäße Verbindungen der Formel (I), worin R¹ Wasserstoff, R² Ethyl und R³ Hydroxy bedeutet und R⁴ die in Tabelle 1 angegebenen Bedeutungen hat:

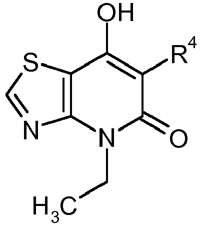

Tabelle 5: Erfindungsgemäße Verbindungen der Formel (I), worin R¹ Wasserstoff, R² Isopropyl und R³ Hydroxy bedeutet, und R⁴ die in Tabelle 1 angegebenen Bedeutungen hat:

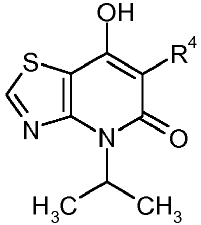

Tabelle 6: Erfindungsgemäße Verbindungen der Formel (I), worin R¹ Wasserstoff, R² Propargyl und R³ Hydroxy bedeutet, und R⁴ die in Tabelle 1 angegebenen Bedeutungen hat:

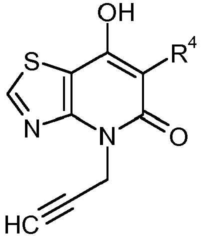

Tabelle 7: Erfindungsgemäße Verbindungen der Formel (I), worin R¹ Wasserstoff, R² Allyl und R³ Hydroxy bedeutet, und R⁴ die in Tabelle 1 angegebenen Bedeutungen hat:

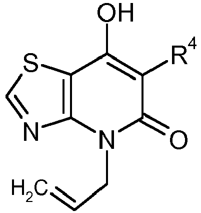

Tabelle 8: Erfindungsgemäße Verbindungen der Formel (I), worin R¹ Wasserstoff, R² Cyanomethyl und R³ Hydroxy bedeutet, und R⁴ die in Tabelle 1 angegebenen Bedeutungen hat:

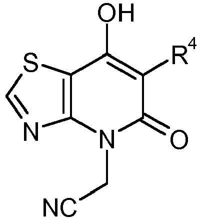

Tabelle 9: Erfindungsgemäße Verbindungen der Formel (I), worin R¹ Wasserstoff, R² Methoxyethyl und R³ Hydroxy bedeutet, und R⁴ die in Tabelle 1 angegebenen Bedeutungen hat:

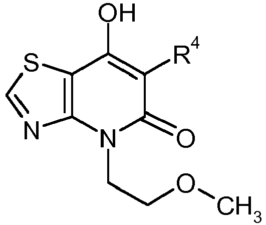

Tabelle 10: Erfindungsgemäße Verbindungen der Formel (I), worin R¹ Wasserstoff, R² Methylthioethyl und R³ Hydroxy bedeutet und R⁴ die in Tabelle 1 angegebenen Bedeutungen hat:

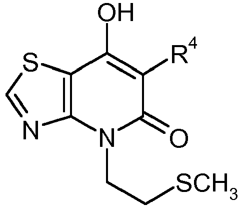

Tabelle 11: Erfindungsgemäße Verbindungen der Formel (I), worin R¹ Methylthio, R² 2,2-Difluoroethyl und R³ Hydroxy bedeutet und R⁴ die in Tabelle 1 angegebenen Bedeutungen hat:

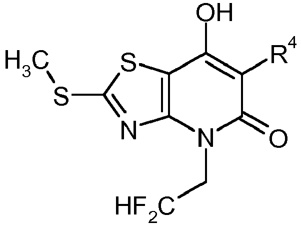

Tabelle 12: Erfindungsgemäße Verbindungen der Formel (I), worin R¹ Methylthio, R² 2,2,2-Trifluoroethyl und R³ Hydroxy bedeutet und R⁴ die in Tabelle 1 angegebenen Bedeutungen hat:

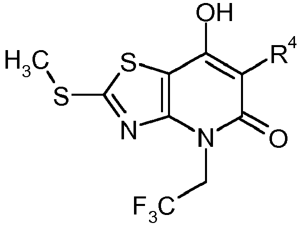

Tabelle 13: Erfindungsgemäße Verbindungen der Formel (I), worin R¹ Methylthio, R² Methyl und R³ Hydroxy bedeutet und R⁴ die in Tabelle 1 angegebenen Bedeutungen hat:

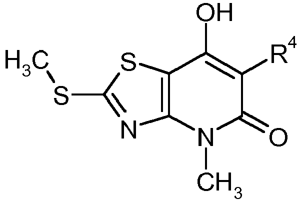

Tabelle 14: Erfindungsgemäße Verbindungen der Formel (I), worin R¹ Methylthio, R² Ethyl und R³ Hydroxy bedeutet und R⁴ die in Tabelle 1 angegebenen Bedeutungen hat:

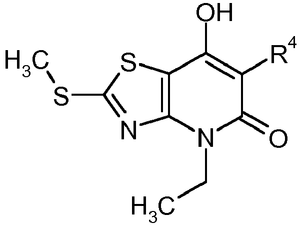

Tabelle 15: Erfindungsgemäße Verbindungen der Formel (I), worin R¹ Methylthio, R² Isopropyl und R³ Hydroxy bedeutet und R⁴ die in Tabelle 1 angegebenen Bedeutungen hat:

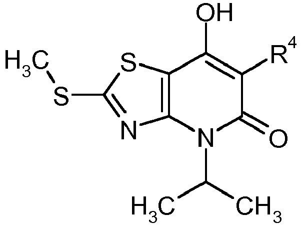

Tabelle 16: Erfindungsgemäße Verbindungen der Formel (I), worin R¹ Methylthio, R² Propargyl und R³ Hydroxy bedeutet und R⁴ die in Tabelle 1 angegebenen Bedeutungen hat:

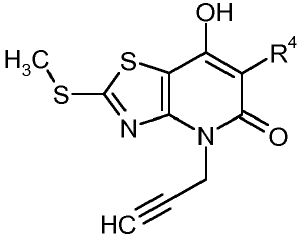

Tabelle 17: Erfindungsgemäße Verbindungen der Formel (I), worin R¹ Methylthio, R² Allyl und R³ Hydroxy bedeutet und R⁴ die in Tabelle 1 angegebenen Bedeutungen hat:

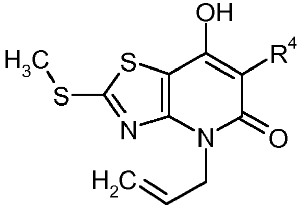

Tabelle 18: Erfindungsgemäße Verbindungen der Formel (I), worin R¹ Methylthio, R² Cyanomethyl und R³ Hydroxy bedeutet und R⁴ die in Tabelle 1 angegebenen Bedeutungen hat:

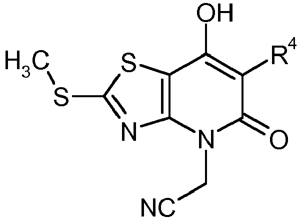

Tabelle 19: Erfindungsgemäße Verbindungen der Formel (I), worin R¹ Methylthio, R² Methoxyethyl und R³ Hydroxy bedeutet und R⁴ die in Tabelle 1 angegebenen Bedeutungen hat:

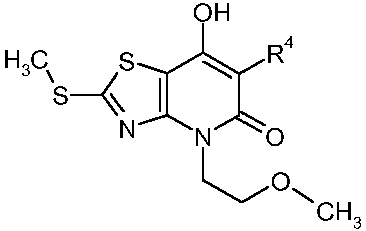

Tabelle 20: Erfindungsgemäße Verbindungen der Formel (I), worin R¹ Methylthio, R² Methylthioethyl und R³ Hydroxy bedeutet und R⁴ die in Tabelle 1 angegebenen Bedeutungen hat:

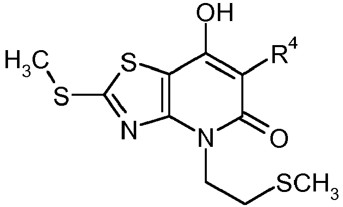

Tabelle 11: Erfindungsgemäße Verbindungen der Formel (I), worin R¹ Methylsulfonyl, R² 2,2-Difluoroethyl und R³ Hydroxy bedeutet und R⁴ die in Tabelle 1 angegebenen Bedeutungen hat:

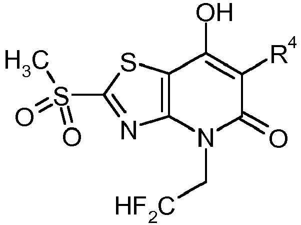

Tabelle 12: Erfindungsgemäße Verbindungen der Formel (I), worin R¹ Methylsulfonyl, R² 2,2,2-Trifluoroethyl und R³ Hydroxy bedeutet und R⁴ die in Tabelle 1 angegebenen Bedeutungen hat:

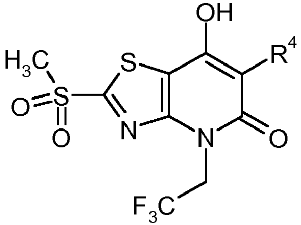

Tabelle 13: Erfindungsgemäße Verbindungen der Formel (I), worin R¹ Methylsulfonyl, R² Methyl und R³ Hydroxy bedeutet und R⁴ die in Tabelle 1 angegebenen Bedeutungen hat:

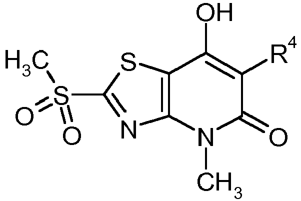

Tabelle 14: Erfindungsgemäße Verbindungen der Formel (I), worin R¹ Methylsulfonyl, R² Ethyl und R³ Hydroxy bedeutet und R⁴ die in Tabelle 1 angegebenen Bedeutungen hat:

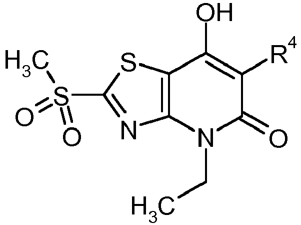

Tabelle 15: Erfindungsgemäße Verbindungen der Formel (I), worin R¹ Methylsulfonyl, R² Isopropyl und R³ Hydroxy bedeutet und R⁴ die in Tabelle 1 angegebenen Bedeutungen hat:

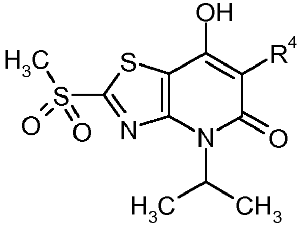

Tabelle 16: Erfindungsgemäße Verbindungen der Formel (I), worin R¹ Methylsulfonyl, R² Propargyl und R³ Hydroxy bedeutet und R⁴ die in Tabelle 1 angegebenen Bedeutungen hat:

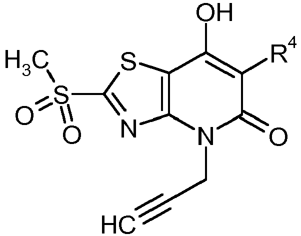

Tabelle 17: Erfindungsgemäße Verbindungen der Formel (I), worin R¹ Methylsulfonyl, R² Allyl und R³ Hydroxy bedeutet und R⁴ die in Tabelle 1 angegebenen Bedeutungen hat:

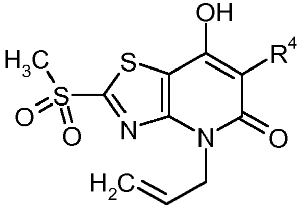

Tabelle 18: Erfindungsgemäße Verbindungen der Formel (I), worin R¹ Methylsulfonyl, R² Benzyl und R³ Hydroxy bedeutet und R⁴ die in Tabelle 1 angegebenen Bedeutungen hat:

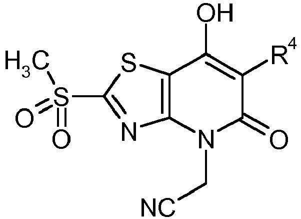

Tabelle 19: Erfindungsgemäße Verbindungen der Formel (I), worin R¹ Methylsulfonyl, R² Methoxyethyl und R³ Hydroxy bedeutet und R⁴ die in Tabelle 1 angegebenen Bedeutungen hat:

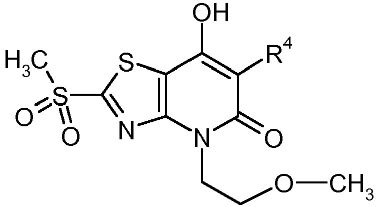

Tabelle 20: Erfindungsgemäße Verbindungen der Formel (I), worin R¹ Methylsulfonyl, R² Methylthioethyl und R³ Hydroxy bedeutet und R⁴ die in Tabelle 1 angegebenen Bedeutungen hat:

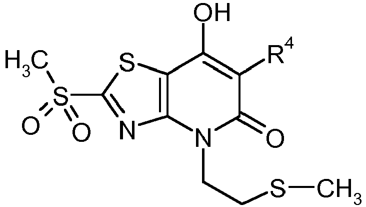

Tabelle 21: Erfindungsgemäße Verbindungen der Formel (I), worin R¹ Methyl, R² 2,2-Difluoroethyl und R³ Hydroxy bedeutet und R⁴ die in Tabelle 1 angegebenen Bedeutungen hat:

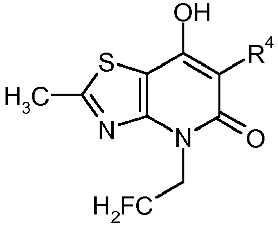

Tabelle 22: Erfindungsgemäße Verbindungen der Formel (I), worin R¹ Methyl, R² 2,2,2-Trifluoroethyl und R³ Hydroxy bedeutet und R⁴ die in Tabelle 1 angegebenen Bedeutungen hat:

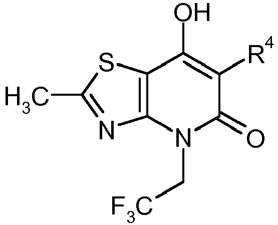

Tabelle 23: Erfindungsgemäße Verbindungen der Formel (I), worin R¹ Methyl, R² Methyl und R³ Hydroxy bedeutet und R⁴ die in Tabelle 1 angegebenen Bedeutungen hat:

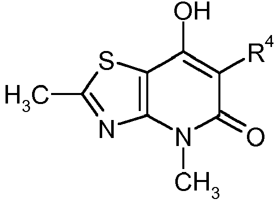

Tabelle 24: Erfindungsgemäße Verbindungen der Formel (I), worin R¹ Methyl, R² Ethyl und R³ Hydroxy bedeutet und R⁴ die in Tabelle 1 angegebenen Bedeutungen hat:

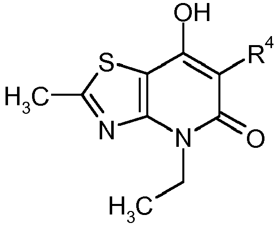

Tabelle 25: Erfindungsgemäße Verbindungen der Formel (I), worin R¹ Methyl, R² Isopropyl und R³ Hydroxy bedeutet und R⁴ die in Tabelle 1 angegebenen Bedeutungen hat:

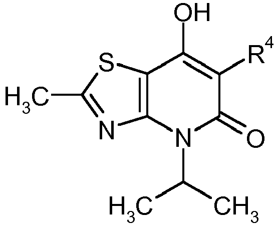

Tabelle 26: Erfindungsgemäße Verbindungen der Formel (I), worin R¹ Methyl, R² Propargyl und R³ Hydroxy bedeutet und R⁴ die in Tabelle 1 angegebenen Bedeutungen hat:

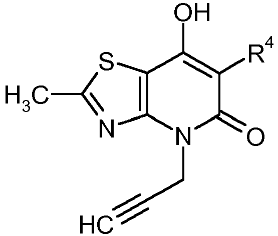

Tabelle 27: Erfindungsgemäße Verbindungen der Formel (I), worin R¹ Methyl, R² Allyl und R³ Hydroxy bedeutet und R⁴ die in Tabelle 1 angegebenen Bedeutungen hat:

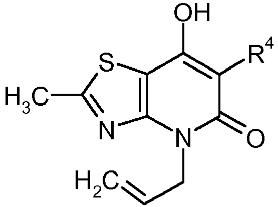

Tabelle 28: Erfindungsgemäße Verbindungen der Formel (I), worin R¹ Methyl, R² Cyanomethyl und R³ Hydroxy bedeutet und R⁴ die in Tabelle 1 angegebenen Bedeutungen hat:

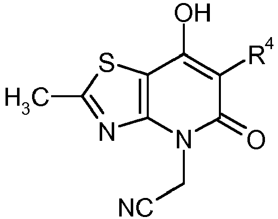

Tabelle 29: Erfindungsgemäße Verbindungen der Formel (I), worin R¹ Methyl, R² Methoxyethyl und R³ Hydroxy bedeutet und R⁴ die in Tabelle 1 angegebenen Bedeutungen hat:

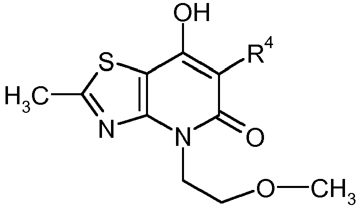

Tabelle 30: Erfindungsgemäße Verbindungen der Formel (I), worin R¹ Methyl, R² Methylthioethyl und R³ Hydroxy bedeutet und R⁴ die in Tabelle 1 angegebenen Bedeutungen hat:

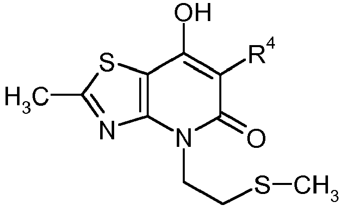

Tabelle 31: Erfindungsgemäße Verbindungen der Formel (I), worin R¹ Cyclopropyl, R² 2,2-Difluoroethyl und R³ Hydroxy bedeutet und R⁴ die in Tabelle 1 angegebenen Bedeutungen hat:

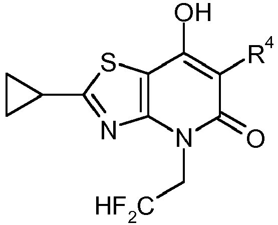

Tabelle 32: Erfindungsgemäße Verbindungen der Formel (I), worin R¹ Cyclopropyl, R² 2,2,2-Trifluoroethyl und R³ Hydroxy bedeutet und R⁴ die in Tabelle 1 angegebenen Bedeutungen hat:

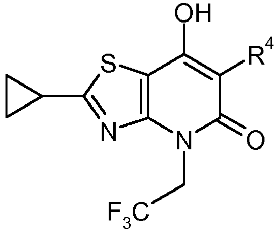

Tabelle 33: Erfindungsgemäße Verbindungen der Formel (I), worin R¹ Cyclopropyl, R² Methyl und R³ Hydroxy bedeutet und R⁴ die in Tabelle 1 angegebenen Bedeutungen hat:

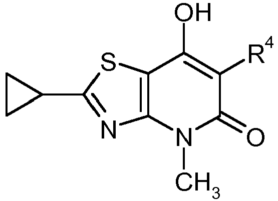

Tabelle 34: Erfindungsgemäße Verbindungen der Formel (I), worin R¹ Cyclopropyl, R² Ethyl und R³ Hydroxy bedeutet und R⁴ die in Tabelle 1 angegebenen Bedeutungen hat:

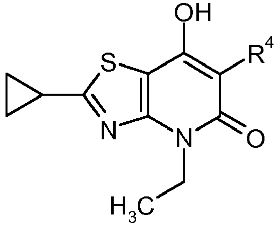

Tabelle 35: Erfindungsgemäße Verbindungen der Formel (I), worin R¹ Cyclopropyl, R² Isopropyl und R³ Hydroxy bedeutet und R⁴ die in Tabelle 1 angegebenen Bedeutungen hat:

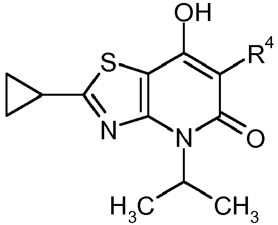

Tabelle 36: Erfindungsgemäße Verbindungen der Formel (I), worin R¹ Cyclopropyl, R² Propargyl und R³ Hydroxy bedeutet und R⁴ die in Tabelle 1 angegebenen Bedeutungen hat:

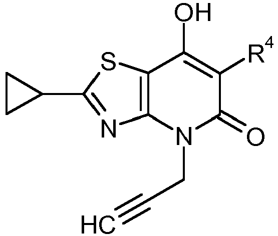

Tabelle 37: Erfindungsgemäße Verbindungen der Formel (I), worin R¹ Cyclopropyl, R² Allyl und R³ Hydroxy bedeutet und R⁴ die in Tabelle 1 angegebenen Bedeutungen hat:

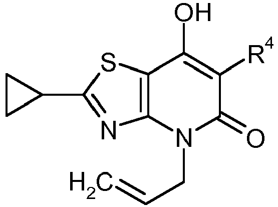

Tabelle 38: Erfindungsgemäße Verbindungen der Formel (I), worin R¹ Cyclopropyl, R² Cyanomethyl und R³ Hydroxy bedeutet und R⁴ die in Tabelle 1 angegebenen Bedeutungen hat:

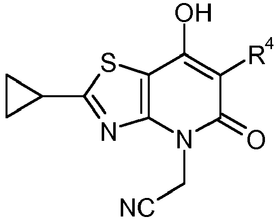

Tabelle 39: Erfindungsgemäße Verbindungen der Formel (I), worin R¹ Cyclopropyl, R² Methoxyethyl und R³ Hydroxy bedeutet und R⁴ die in Tabelle 1 angegebenen Bedeutungen hat:

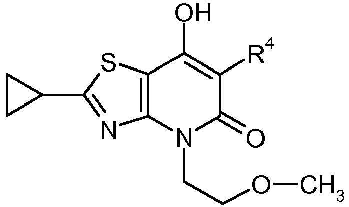

Tabelle 40: Erfindungsgemäße Verbindungen der Formel (I), worin R¹ Cyclopropyl, R² Methylthioethyl und R³ Hydroxy bedeutet und R⁴ die in Tabelle 1 angegebenen Bedeutungen hat:

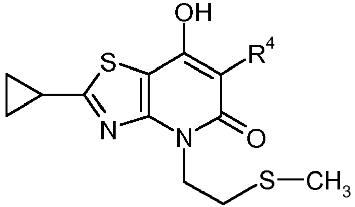

Tabelle 41: Erfindungsgemäße Verbindungen der Formel (I), worin R¹ Methoxy, R² 2,2-Difluoroethyl und R³ Hydroxy bedeutet und R⁴ die in Tabelle 1 angegebenen Bedeutungen hat:

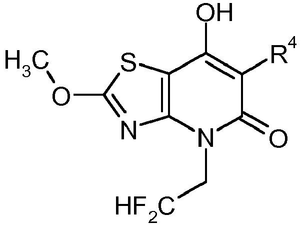

Tabelle 42: Erfindungsgemäße Verbindungen der Formel (I), worin R¹ Methoxy, R² 2,2,2-Trifluoroethyl und R³ Hydroxy bedeutet und R⁴ die in Tabelle 1 angegebenen Bedeutungen hat:

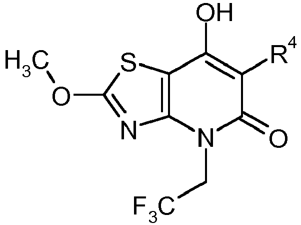

Tabelle 43: Erfindungsgemäße Verbindungen der Formel (I), worin R¹ Methoxy, R² Methyl und R³ Hydroxy bedeutet und R⁴ die in Tabelle 1 angegebenen Bedeutungen hat:

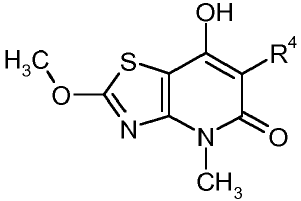

Tabelle 44: Erfindungsgemäße Verbindungen der Formel (I), worin R¹ Methoxy, R² Ethyl und R³ Hydroxy bedeutet und R⁴ die in Tabelle 1 angegebenen Bedeutungen hat:

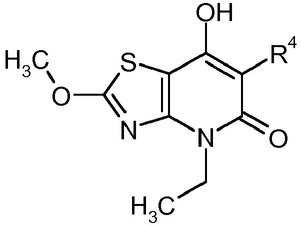

Tabelle 45: Erfindungsgemäße Verbindungen der Formel (I), worin R¹ Methoxy, R² Isopropyl und R³ Hydroxy bedeutet und R⁴ die in Tabelle 1 angegebenen Bedeutungen hat:

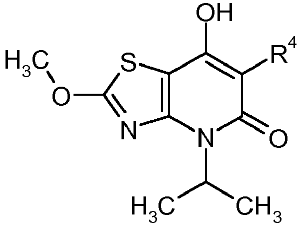

Tabelle 46: Erfindungsgemäße Verbindungen der Formel (I), worin R¹ Methoxy, R² Propargyl und R³ Hydroxy bedeutet und R⁴ die in Tabelle 1 angegebenen Bedeutungen hat:

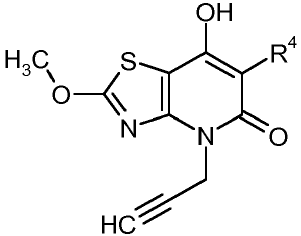

Tabelle 47: Erfindungsgemäße Verbindungen der Formel (I), worin R¹ Methoxy, R² Allyl und R³ Hydroxy bedeutet und R⁴ die in Tabelle 1 angegebenen Bedeutungen hat:

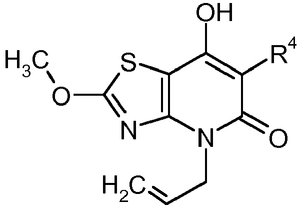

Tabelle 48: Erfindungsgemäße Verbindungen der Formel (I), worin R¹ Methoxy, R² Cyanomethyl und R³ Hydroxy bedeutet und R⁴ die in Tabelle 1 angegebenen Bedeutungen hat:

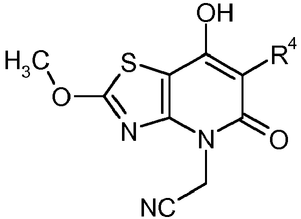

Tabelle 49: Erfindungsgemäße Verbindungen der Formel (I), worin R¹ Methoxy, R² Methoxyethyl und R³ Hydroxy bedeutet und R⁴ die in Tabelle 1 angegebenen Bedeutungen hat:

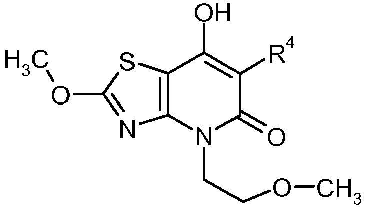

Tabelle 50: Erfindungsgemäße Verbindungen der Formel (I), worin R¹ Methoxy, R² Methylthioethyl und R³ Hydroxy bedeutet und R⁴ die in Tabelle 1 angegebenen Bedeutungen hat:

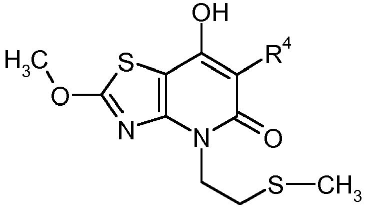

Tabelle 51: Erfindungsgemäße Verbindungen der Formel (I), worin R¹ Ethoxy, R² 2,2-Difluoroethyl und R³ Hydroxy bedeutet und R⁴ die in Tabelle 1 angegebenen Bedeutungen hat:

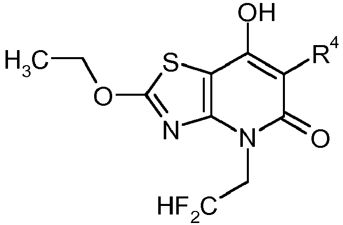

Tabelle 52: Erfindungsgemäße Verbindungen der Formel (I), worin R¹ Ethoxy, R² 2,2,2-Trifluoroethyl und R³ Hydroxy bedeutet und R⁴ die in Tabelle 1 angegebenen Bedeutungen hat:

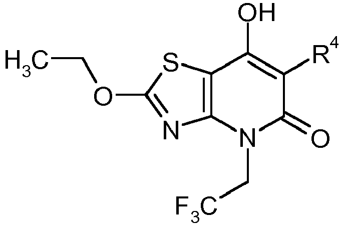

Tabelle 53: Erfindungsgemäße Verbindungen der Formel (I), worin R¹ Ethoxy, R² Methyl und R³ Hydroxy bedeutet und R⁴ die in Tabelle 1 angegebenen Bedeutungen hat:

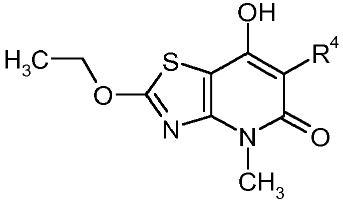

Tabelle 54: Erfindungsgemäße Verbindungen der Formel (I), worin R¹ Ethoxy, R² Ethyl und R³ Hydroxy bedeutet und R⁴ die in Tabelle 1 angegebenen Bedeutungen hat:

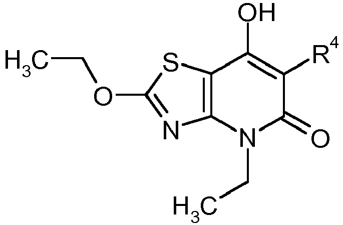

Tabelle 55: Erfindungsgemäße Verbindungen der Formel (I), worin R¹ Ethoxy, R² Isopropyl und R³ Hydroxy bedeutet und R⁴ die in Tabelle 1 angegebenen Bedeutungen hat:

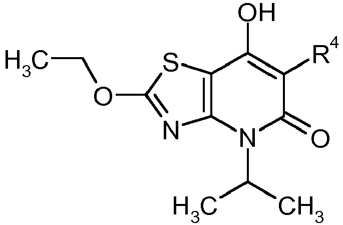

Tabelle 56: Erfindungsgemäße Verbindungen der Formel (I), worin R¹ Ethoxy, R² Propargyl und R³ Hydroxy bedeutet und R⁴ die in Tabelle 1 angegebenen Bedeutungen hat:

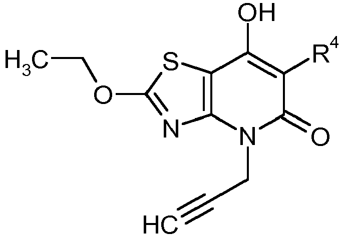

Tabelle 57: Erfindungsgemäße Verbindungen der Formel (I), worin R¹ Ethoxy, R² Allyl und R³ Hydroxy bedeutet und R⁴ die in Tabelle 1 angegebenen Bedeutungen hat:

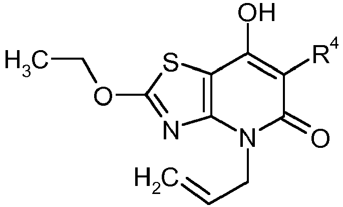

Tabelle 58: Erfindungsgemäße Verbindungen der Formel (I), worin R¹ Ethoxy, R² Cyanomethyl und R³ Hydroxy bedeutet und R⁴ die in Tabelle 1 angegebenen Bedeutungen hat:

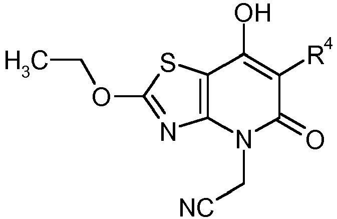

Tabelle 59: Erfindungsgemäße Verbindungen der Formel (I), worin R¹ Ethoxy, R² Methoxyethyl und R³ Hydroxy bedeutet und R⁴ die in Tabelle 1 angegebenen Bedeutungen hat:

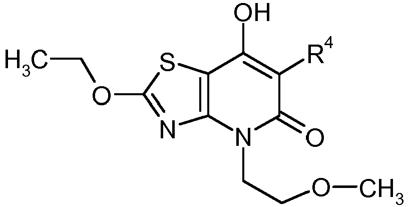

Tabelle 60: Erfindungsgemäße Verbindungen der Formel (I), worin R¹ Ethoxy, R² Methylthioethyl und R³ Hydroxy bedeutet und R⁴ die in Tabelle 1 angegebenen Bedeutungen hat:

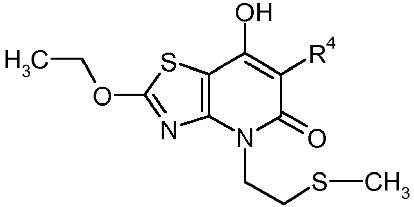

Tabelle 61: Erfindungsgemäße Verbindungen der Formel (I), worin R¹ Methoxyethoxy, R² 2,2-Difluoroethyl und R³ Hydroxy bedeutet und R⁴ die in Tabelle 1 angegebenen Bedeutungen hat:

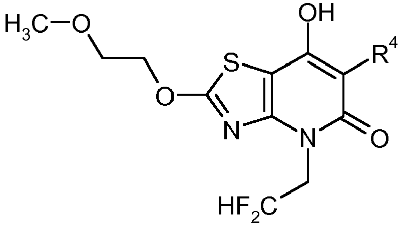

Tabelle 62: Erfindungsgemäße Verbindungen der Formel (I), worin R¹ Methoxyethoxy, R² 2,2,2-Trifluoroethyl und R³ Hydroxy bedeutet und R⁴ die in Tabelle 1 angegebenen Bedeutungen hat:

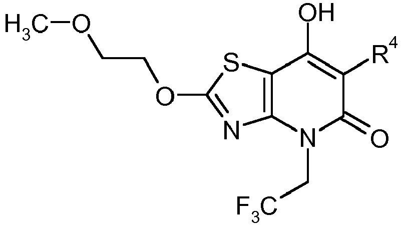

Tabelle 63: Erfindungsgemäße Verbindungen der Formel (I), worin R¹ Methoxyethoxy, R² Methyl und R³ Hydroxy bedeutet und R⁴ die in Tabelle 1 angegebenen Bedeutungen hat:

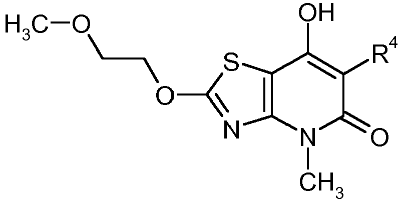

Tabelle 64: Erfindungsgemäße Verbindungen der Formel (I), worin R¹ Methoxyethoxy, R² Ethyl und R³ Hydroxy bedeutet und R⁴ die in Tabelle 1 angegebenen Bedeutungen hat:

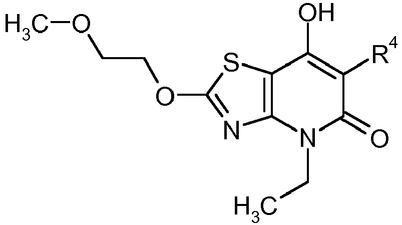

Tabelle 65: Erfindungsgemäße Verbindungen der Formel (I), worin R¹ Methoxyethoxy, R² Isopropyl und R³ Hydroxy bedeutet und R⁴ die in Tabelle 1 angegebenen Bedeutungen hat:

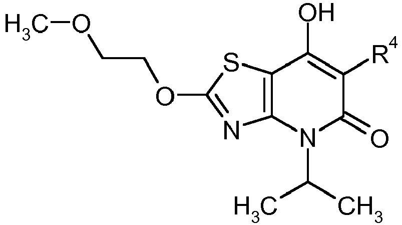

Tabelle 66: Erfindungsgemäße Verbindungen der Formel (I), worin R¹ Methoxyethoxy, R² Propargyl und R³ Hydroxy bedeutet und R⁴ die in Tabelle 1 angegebenen Bedeutungen hat:

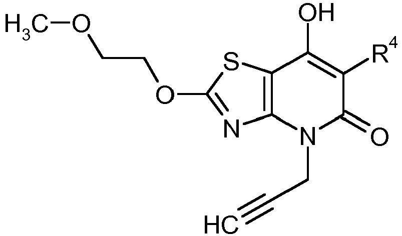

Tabelle 67: Erfindungsgemäße Verbindungen der Formel (I), worin R¹ Methoxyethoxy, R² Allyl und R³ Hydroxy bedeutet und R⁴ die in Tabelle 1 angegebenen Bedeutungen hat:

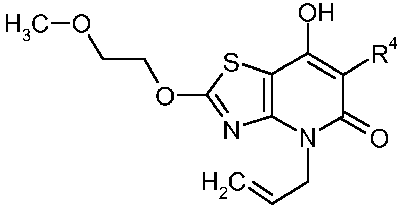

Tabelle 68: Erfindungsgemäße Verbindungen der Formel (I), worin R¹ Methoxyethoxy, R² Cyanomethyl und R³ Hydroxy bedeutet und R⁴ die in Tabelle 1 angegebenen Bedeutungen hat:

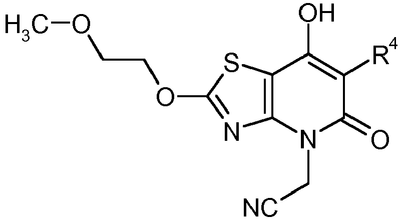

Tabelle 69: Erfindungsgemäße Verbindungen der Formel (I), worin R¹ Methoxyethoxy, R² Methoxyethyl und R³ Hydroxy bedeutet und R⁴ die in Tabelle 1 angegebenen Bedeutungen hat:

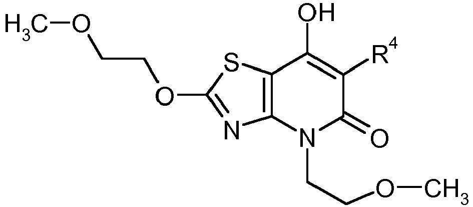

Tabelle 70: Erfindungsgemäße Verbindungen der Formel (I), worin R¹ Methoxyethoxy, R² Methylthioethyl und R³ Hydroxy bedeutet und R⁴ die in Tabelle 1 angegebenen Bedeutungen hat:

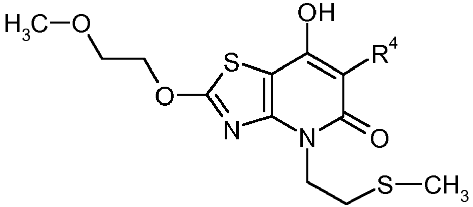

Erfindungsgemäße Verbindungen der Formel (I), worin R³ für Wasserstoff steht, können beispielsweise gemäß der in Schema 1 angegebenen Methode durch baseninduzierte Kondensationsreaktion von Verbindungen der Formel (II) hergestellt werden. Darin steht R⁷ für (C₁-C₆)-Alkyl, insbesonders für Methyl oder Ethyl.

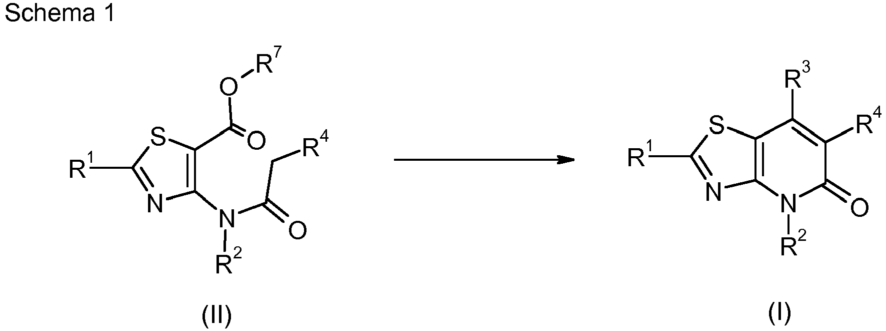

Verbindungen der Formel (II) lassen sich beispielsweise gemäß der in Schema 1a angegebenen Methoden durch Reaktion von Aminocarbonsäurederivaten mit Phenylessigsäurederivaten herstellen. Darin steht U für eine durch Carbonsäureaktivierungsreagenzien, wie Carbonyldiimidazol, Carbonyldiimide (wie z.B. Dicyclohexylcarbondiimid), Phosphorylierungsreagenzien (wie z.B. POCl₃, BOP-CI), Halogenierungsmittel wie z.B. Thionylchlorid, Oxalylchlorid, Phosgen oder Chlorameisensäureester eingeführte Abgangsgruppe. Solche Methoden sind auch aus WO2008/009908 A1 und WO2008/071918 A1 bzw. WO2009/063180 und dort zitierten Dokumenten dem Fachmann bekannt. Verbindungen Verbindungen der Formel (II) sind neu und ebenfalls ein Gegenstand der vorliegenden Erfindung.

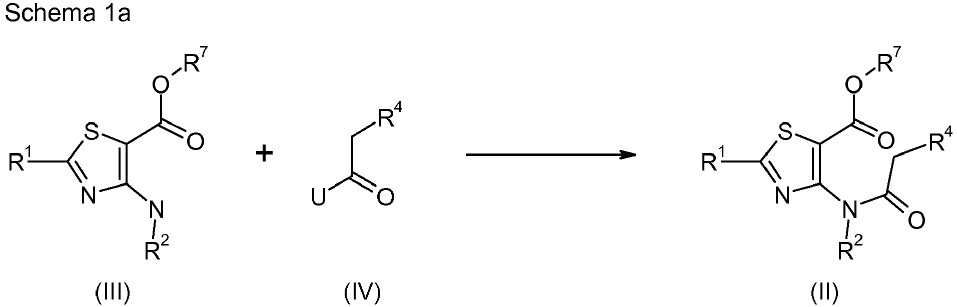

Die zur Herstellung der in Schema 1a genannten Phenylessigsäurederivaten notwendigen freien Phenylessigsäuren, d.h. solche worin U für Hydroxy steht, sind bekannt oder lassen sich nach an sich und beispielsweise aus WO 2005/075401, WO 2001/96277, WO 1996/35664 und WO 1996/25395 bekannten Verfahren herstellen. Für den Fall, dass R² einen anderen Rest als Wasserstoff bedeuten soll, kann ein Rest R^{2'} nach literaturbekannten Methoden eingeführt werden beispielsweise über reduktive Aminierung eines entsprechenden Aminosäureesters mit einem Aldehyd gefolgt von einer Reduktion beispielsweise mit Natriumcyanoborhydrid. R^{2'} bedeutet jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₄)-Alkoxy-(C₁-C₆)-alkyl, Di-(C₁-C₄)-alkoxy-(C₁-C₆)-alkyl oder (C₁-C₄)-Alkylthio-(C₁-C₆)-alkyl, (C₃-C₆)-Cycloalkyl-(C₁-C₆)-alkyl.

Weiterhin ist bekannt, dass Umsetzungen mit R^{2'}-Halogeniden oder auch entsprechenden Sulfonaten mit entsprechenden Aminosäureestern zu den gewünschten Vorstufen führen. Alternativ kann entsprechend auch nach erfolgter Kondensation des Aminosäureesters mit der entsprechenden Phenylessigsäure die Alkylierung mit R^{2'}-Halogeniden oder Sulfonaten erfolgen (s. Schema 1 b), die dann ebenfalls zu den erfindungsgemäßen Zwischenprodukten (II) führt.

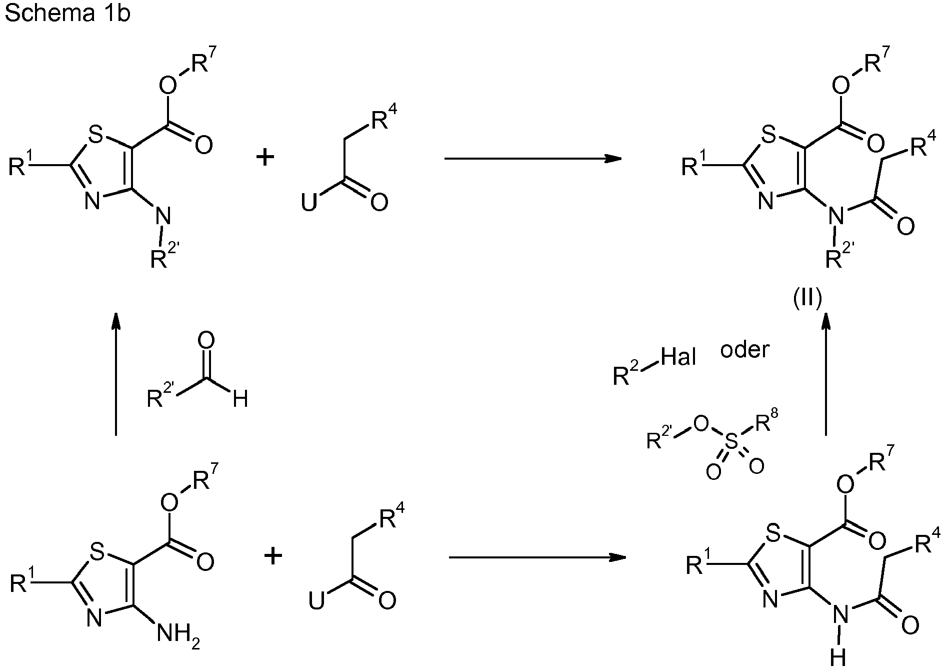

Bestimmte Phenylessigsäurederivate lassen sich auch unter Verwendung von Essigesterenolaten in Gegenwart von Palladiumkatalysatoren, z.B. gebildet aus einer Palladiumquelle (z.B. Pd₂(dba)₃ oder Pd(Oac)₂) und einem Liganden (z.B. (t-Bu)₃P, iMes*HCl oder 2'-(N,N-Dimethylamino)-2-(dicyclohexylphosphanyl)biphenyl) hergestellt (WO 2005/048710, J. Am. Chem. Soc 2002. 124,. 12557, J. Am. Chem. Soc 2003. 125, 11176 oder J. Am. Chem. Soc. 2001, 123, 799) herstellen. Darüber hinaus lassen sich bestimmte substituierte Arylhalogenide unter Kupferkatalyse in die korrespondierenden substituierten Malonester überführen (z.B. beschrieben in Org. Lett. 2002, 2, 269, WO 2004/108727), welche nach bekannten Methoden in Phenylessigsäuren überführt werden können.
Erfindungsgemäße Verbindungen der Formel (I), worin R³ für OH steht, können beispielsweise auch gemäß der in Schema 2 angegebenen Methode durch Reaktion von Verbindungen der Formel (I), worin R³ für Alkoxy, bevorzugt für Methoxy, steht, mit starken Mineralbasen, wie Natrium- oder Kaliumhydroxid, oder in konzentrierten Mineralsäuren, wie Bromwassserstoffsäure, hergestellt werden.

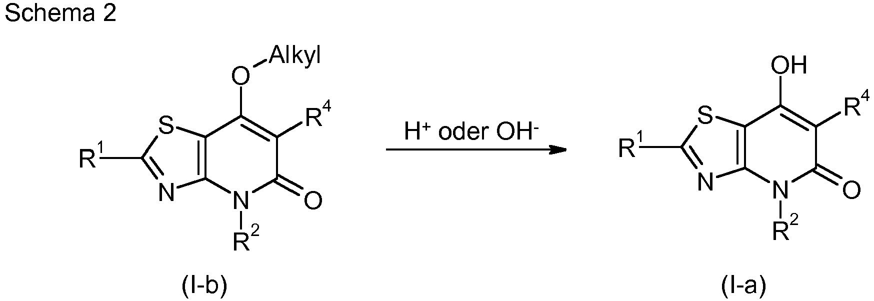

Erfindungsgemäße Verbindungen der Formel (I), worin R³ für O-C(=O)R⁸ steht, können beispielsweise durch dem Fachmann bekannte Reaktionen von Verbindungen der Formel (I), worin R³ für Wasserstoff steht, mit Carbonsäurehalogeniden der Formel Hal-CO-R⁸ oder mit Carbonsäureanhydriden der Formel R⁸-CO-O-CO-R⁸ hergestellt werden.

Erfindungsgemäße Verbindungen der Formel (I), worin R³ für O-C(=L)MR⁹ steht, können beispielsweise durch dem Fachmann bekannte Reaktionen von Verbindungen der Formel (I), worin R³ für Wasserstoff steht, mit a) Chlorameisensäureestern oder Chlorameisensäurethioestern der Formel R⁹-M-COOR⁸ oder b) mit Chlorameisensäurehalogeniden oder Chlorameisensäurethiohalogeniden hergestellt werden.

Erfindungsgemäße Verbindungen der Formel (I), worin R³ für SO₂R¹⁰ steht, können beispielsweise durch dem Fachmann bekannte Reaktionen von Verbindungen der Formel (I), worin R³ für Wasserstoff steht, mit Sulfonsäurechloriden der Formel R¹⁰-SO₂-Cl hergestellt werden.

Erfindungsgemäße Verbindungen der Formel (I), worin R³ für P(=L)R¹¹R¹² steht, können beispielsweise durch dem Fachmann bekannte Reaktionen von Verbindungen der Formel (I), worin R³ für Wasserstoff steht, mit Phosphorsäurechloriden der Formel Hal-P(=L)R¹¹R¹² hergestellt werden.

Erfindungsgemäße Verbindungen der Formel (I), worin R³ für E steht, können beispielsweise durch dem Fachmann bekannte Reaktionen von Verbindungen der Formel (I), worin R³ für Wasserstoff steht, mit Metallverbindungen der Formel Met(OR¹³)ₜ oder mit Aminen hergestellt werden. Darin bedeutet Met ein ein- oder zweiwertiges Metallion, bevorzugt ein Alkali- oder Erdalkalimetall wie Lithium, Natrium, Kalium, Magnesium oder Calcium. Der Index t steht für 1 oder 2. Ein Ammoniumion bedeutet die Gruppe NH₄⁺ oder R¹⁴R¹⁵R¹⁶R¹⁷N⁺, worin R¹⁴, R¹⁵, R¹⁶ und R¹⁷ unabhängig voneinander vorzugsweise (C₁-C₆)-Alkyl oder Benzyl bedeuten. Erfindungsgemäße Verbindungen der Formel (I), worin R³ für C(=L)NR¹⁸R¹⁹ steht, können beispielsweise durch dem Fachmann bekannte Reaktionen von Verbindungen der Formel (I), worin R³ für Wasserstoff steht, mit Isocyanaten oder Isothiocyanaten der Formel R¹⁸-N=C=L oder mit Carbamidsäurechloriden oder Thiocarbamidsäurechloriden der Formel R¹⁸R¹⁹N-C(=L)Cl hergestellt werden. Erfindungsgemäße Verbindungen der Formel (I), worin R³ für Alkoxy, bevorzugt für Methoxy, steht, können beispielsweise auch gemäß Schema 3 durch dem Fachmann bekannte Reaktionen von Verbindungen der Formel (I-c) mit Verbindungen der Formel (V) hergestellt werden. Darin steht L für Brom oder Iod, und Q bedeutet eine Trialkylzinngruppe, eine Magnesiumhalogenidgruppe oder bevorzugt eine Boronsäure oder deren Ester. Diese Reaktionen werden üblicherweise in Gegenwart eines Katalysators (z. B. Pd-Salze oder Pd-Komplexe) und in Gegenwart einer Base (z.B. Natriumcarbonat, Kaliumphosphat) durchgeführt.

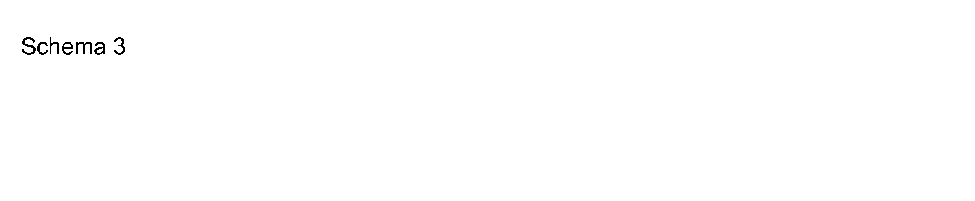

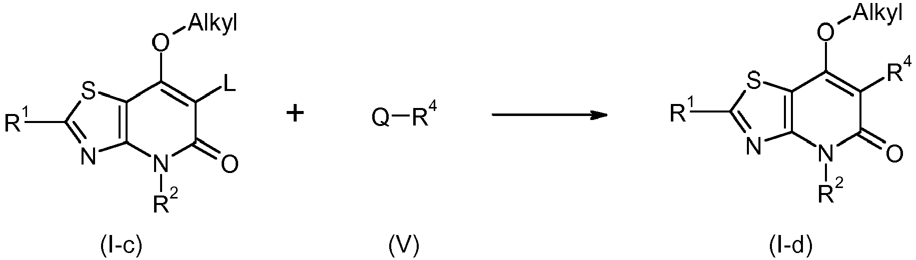

Die Verbindungen der allgemeinen Formel (II-c) können durch Umsetzung der Verbindungen der allgemeinen Formel (II-b) mit (Het)Aryl-Boronsäuren oder (Het)Aryl-Boronsäureestern unter Suzuki-Bedingungen mit einer katalytischen Menge eines Übergangsmetall-Katalysators, vorzugsweise eines PalladiumKatalysators- wie z.B. Palladium(II)acetat und einer Ligand-Verbindung, vorzugsweise Trialkylphosphin oder Triarylphosphin, wie Tricyclohexylphosphin und einer Base, vorzugsweise Kalium-tert-Butylat, Trialkylamine oder Kalium- oder Caesiumcarbonat oder Kaliumphosphat in einem inerten Lösungsmittel (z. B. Toluol oder Dimethylformamid ggf. jeweils im Gemisch mit Wasser (3:1)) erhalten werden bei Reaktionstemperaturen zwischen -20°C und der Rückflusstemperatur des Lösemittels (Schema 4).

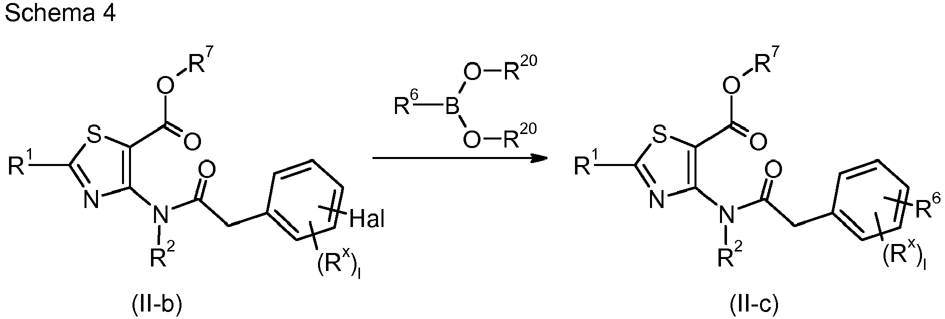

Die Aufarbeitung der jeweiligen Reaktionsmischungen erfolgt in der Regel nach bekannten Verfahren, beispielsweise durch Kristallisation, wässrig-extraktive Aufarbeitung, durch chromatographische Methoden oder durch Kombination dieser Methoden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die erfindungsgemäßen Verbindungen zeigen auch hohe insektizide Wirkungen. Ein weiterer Gegenstand vorliegender Erfindung sind somit insektizide Mittel enthaltend Verbindungen der Formel (I).

Die nachstehenden Beispiele erläutern die Erfindung.

### A. Chemische Beispiele

### 1. Herstellung von 6-(4'-Chlor-4-methylbiphenyl-3-yl)-7-hydroxy-4-(prop-2-in-1-yl)[1,3]thiazolo[4,5-b]pyridin-5(4H)-on (Verbindung I-a-40)

### 1.1. Herstellung von Methyl-4-{[(4'-chlor-4-methylbiphenyl-3-yl)acetyl]amino}-1,3-th iazol-5-carboxylat

1.8 g (11.38 mmol) Methyl-4-amino-1,3-thiazol-5-carboxylat wurden in 40 Acetonitril gelöst und mit 1.8 ml (22.76 mmol) Pyridin versetzt. Nach 5 min Rühren bei Raumtemperatur (RT) wurde eine Lösung von 3.18 g (11.38 mmol) (4'Chlor-4-methylbiphenyl-3-yl)acetylchlorid (erhalten aus (4'-Chlor-4-methylbiphenyl-3-yl)essigsäure gemäß WO 99/48869 A1) in 20 ml Acetonitril zugetropft. Die Mischung wurde 12 h bei RT gerührt. Der entstandene Niederschlag wurde abfiltriert, mit Wasser gewaschen und vakuumgetrocknet. Die entstandene Mutterlauge wurde eingedampft, in Chloroform gelöst und chromatographiert (Ethylacetat/n-Heptan: 20/80 nach 80/20). Man erhält insgesamt 1.81 g Methyl-4-{[(4'-chlor-4-methylbiphenyl-3-yl)acetyl]amino}-1,3-thiazol-5-carboxylat.
¹HNMR (400 MHz, CDCl₃): 9.78 (br s, 1 H), 8.84 (s, 1 H), 7.58 (m, 2H), 7.52 (m, 1 H), 7.44 (m, 1 H), 7.40 (m, 2H), 7.32 (m, 1 H), 3.95 (br s, 2H), 3.76 (s, 3H), 2.42 (s, 3H).

### 1.2. Herstellung von Methyl-4-{[(4'-chlor-4-methylbiphenyl-3-yl)acetyl](prop-2-in-1-yl)amino}-1,3-thiazol-5-carboxylat

600 mg (1.5 mmol) Methyl-4-{[(4'-chlor-4-methylbiphenyl-3-yl)acetyl]amino}-1,3-thiazol-5-carboxylat wurden in 20 ml Acetonitril gelöst und mit 1.2 g (3.7 mmol) Cäsiumcarbonat, 449 mg (3 mmol) Natriumiodid und 0.33 ml (3 mmol, 80%ig) Propargylbromid versetzt. Anschließend wurde 12 bei RT gerührt. Die entstandene Suspension wurde filtriert. Die Mutterlauge wurde eingedampft. Der Rückstand wurde mit Wasser aufgenommen und die Lösung mit Dichlormethan extrahiert. Die organische Phase wurde getrocknet und eingedampft. Der Rückstand wurde in Acetonitril aufgenommen und chromatographiert (Acetonitril/Wasser (0,05 % Trifluoressigsäure 20/80 nach 100/0)). Man erhielt 415 mg Methyl-4-{[(4'-chlor-4-methylbiphenyl-3-yl)acetyl](prop-2-in-1-yl)amino}-1,3-thiazol-5-carboxylat.
¹HNMR (400 MHz, CDCl₃): 8.82 (s, 1 H), 7.45 (m, 2H), 7.37 (m, 2H), 7.29 (m, 2H), 7.14 (m, 1 H), 7.07 (br s, 1 H), 4.64 (br d, 2H), 3.78 (s, 3H), 3.64 (br s, 2H), 2.20 (s, 3H), 2.14 (br s, 1 H).

### 1.3 Herstellung von 6-(4'-Chlor-4-methylbiphenyl-3-yl)-7-hydroxy-4-(prop-2-in-1-yl)[1,3]thiazolo[4,5-b]pyridin-5(4H)-on (Verbindung I-a-40)

415 mg (0.95 mmol) Methyl-4-{[(4'-chlor-4-methylbiphenyl-3-yl)acetyl](prop-2-in-1-yl)amino}-1,3-thiazol-5-carboxylat wurden in 15 ml THF gelöst und bei RT mit 34 mg (1.4 mmol) NaH versetzt. Die Reaktionsmischung wurde 4 h bei RT gerührt. Dann wurde das THF unter Vakuum entfernt und der Rückstand in Wasser aufgenommen. Die wässrige Lösung wurde mit Dichlormethan gewaschen und danach mit 1 N Salzsäure angesäuert. Danach wurde mit Dichlormethan extrahiert, die organische Phase getrocknet und eingedampft. Man erhielt 397 mg 6-(4'-Chlor-4-methylbiphenyl-3-yl)-7-hydroxy-4-(prop-2-in-1-yl)[1,3]thiazolo[4,5-b]pyridin-5(4H)-on.

### 2. Herstellung von O-[6-(4'-Chlor-4-methylbiphenyl-3-yl)-5-oxo-4-(prop-2-in-1-yl)-4,5-dihydro[1,3]thiazolo[4,5-b]pyridin-7-yl]-S-methylthiocarbonat (Verbindung I-a-41)

150 mg (0.37 mmol) 6-(4'-Chlor-4-methylbiphenyl-3-yl)-7-hydroxy-4-(prop-2-in-1-yl)[1,3]thiazolo[4,5-b]pyridin-5(4H)-on wurden in 15 ml Dichlormethan gelöst, mit 0.05 ml (0.55 mmol) Pyridin und mit 1.3 ml (0.44 mmol) Chlorameisensäure-S-methylester versetzt. Danach wurde 5 h bei RT gerührt. Das Dichlormethan wurde dann im Vakuum entfernt, und der Rückstand wurde mit Wasser aufgenommen. Die wässrige Lösung wurde mit Dichlormethan extrahiert, die organische Phase getrocknet und eingedampft. Chromatographie (Ethylacetat/n-Heptan: 20/80 nach 80/20) lieferte 114 mg O-[6-(4'-Chlor-4-methylbiphenyl-3-yl)-5-oxo-4-(prop-2-in-1-yl)-4,5-dihydro[1,3]thiazolo[4,5-b]pyridin-7-yl]-S-methylthiocarbonat.

3. Herstellung von 6-(4'-Chlor-4-methylbiphenyl-3-yl)-5-oxo-4-(prop-2-in-1-yl)-4,5-dihydro[1,3]thiazolo[4,5]pyridin-7-yl-2-methylpropanoat (Verbindung I-a-42) 150 mg (0.37 mmol) 6-(4'-Chlor-4-methylbiphenyl-3-yl)-7-hydroxy-4-(prop-2-in-1-yl)[1,3]thiazolo[4,5-b]pyridin-5(4H)-on wurden in 15 ml Dichlormethan gelöst, mit 0.05 ml (0.55 mmol) Pyridin und anschließend mit 47.1 mg (0.44 mmol) 2-Methylpropionylchlorid versetzt. Nach 4 h Rühren bei RT wurde die Reaktionslösung mit 2N Salzsäure gewaschen und eingedampft. Der Rückstand wurde chromatographiert: (Acetonitril/Wasser (0,05% Trifluoressigsäure 20/80 nach 100/0)). Man erhielt 76 mg von 6-(4'-Chlor-4-methylbiphenyl-3-yl)-5-oxo-4-(prop-2-in-1-yl)-4,5-dihydro[1,3]thiazolo[4,5]pyridin-7-yl-2-methylpropanoat.

### 4. Herstellung von 6-[2,6-Dichlor-3-(5-chlor-2-thienyl)phenyl]-4-(2,2-difluorethyl)-7-hydroxy[1,3]thiazolo[4,5-b]pyridin-5(4H)-on (Verbindung I-a-14)

4.1. Herstellung von Methyl-4-{[(3-brom-2,6-dichlorphenyl)acetyl]amino}-1,3-th iazol-5-carboxylat
2.0 g (12.64 mmol) Methyl-4-amino-1,3-thiazol-5-carboxylat wurden in 20 Acetonitril gelöst und mit 2.05 ml (25.29 mmol) Pyridin versetzt. Nach 5 min Rühren bei Taumtemperatur wurde eine Lösung von 4.21 g (13.91 mmol) (3-Brom-2,6-dichlorphenyl)acetylchlorid (erhalten aus (3-Brom-2,6-dichlorphenyl)essigsäure, siehe WO9736868) in 10 ml Acetonitril zugetropft. Die Reaktionsmischung wurde 12 h bei RT gerührt. Der entstandene Niederschlag wurde abfiltriert, mit Wasser gewaschen und vakuumgetrocknet. Die entstandene Mutterlauge wurde eingedampft, in Chloroform gelöst und chromatographiert (Ethylacetat/n-Heptan: 20/80 nach 80/20). Man erhält insgesamt 4.01 g Methyl-4-{[(3-brom-2,6-dichlorphenyl)acetyl]amino}-1,3-thiazol-5-carboxylat.
¹HNMR (400 MHz, DMSO-d₆): 10.71 (br s, 1 H), 9.22 (s, 1 H), 7.76 (d, 1 H), 7.47 (d, 1 H), 4.23 (s, 2H), 3.76 (s, 3H).

### 4.2. Herstellung von Methyl-4-({[2,6-dichlor-3-(5-chlor-2-thienyl)phenyl]acetyl} amino)-1,3-thiazol-5-carboxylat

300 mg (0.71 mmol) Methyl-4-{[(3-brom-2,6-dichlorphenyl)acetyl]amino}-1,3-thiazol-5-carboxylat und 230 mg (1.41 mmol) (5-Chlor-2-thienyl)boronsäure wurden in einer Mischung von 18 ml Toluol und 3 ml Wasser gelöst und nacheinander mit 450 mg (2.12 mmol) Kaliumphosphat, 15.9 mg (0.07 mmol) Palladiumacetat und 20 mg (0.07 mmol) Tricyclohexylphosphin versetzt. Anschließend wurde das Reaktionsgemisch 1 h bei 180°C unter Mikrowelleneinwirkung erhitzt. Das Gemisch wurde filtriert und das Filtrat in Ethylacetat und Wasser aufgenommen. Die organische Phase wurde getrocknet und eingedampft. Es wurden 170 mg Methyl-4-({[2,6-dichlor-3-(5-chlor-2-thienyl)phenyl]acetyl} amino)-1,3-thiazol-5-carboxylat erhalten, die ohne weitere Aufreinigung weiter umgesetzt wurden.
¹HNMR (400 MHz, CDCl₃): 9.80 (br s, 1 H), 8.83 (s, 1 H), 7.57 (m, 1 H), 7.25 (m, 1 H), 6.85 (d, 1 H), 6.83 (d, 1 H), 4.42 (br s, 2H), 3.87 (s, 3H).

### 4.3. Herstellung von Methyl-4-[{[2,6-dichlor-3-(5-chlor-2-thienyl)phenyl]acetyl}(2,2-difluorethyl)amino]-1,3-thiazol-5-carboxylat

In einer Argon-Atmosphäre wurden 300 mg (0,65 mmol) Methyl-4-({[2,6-dichlor-3-(5-chlor-2-thienyl)phenyl]acetyl} amino)-1,3-thiazol-5-carboxylat in 15 ml Tetrahydrofuran gelöst, bei Raumtemperatur 23 mg (0.97 mmol) Natriumhydrid zugegeben. Die Reaktionsmischung wurde 5 min bei RT gerührt und anschließend innerhalb 10 min mit einer Lösung von 167 mg (0,78 mmol) 2,2-Difluorethyltrifluormethansulfonat in 6 ml THF versetzt. Danach wird 12 h bei RT gerührt. Das THF wird unter Vakuum abgezogen und der Rückstand mit Wasser aufgenommen. Die wässrige Phase wird mit Dichlormethan extrahiert, die organische Phase getrocknet und eingedampft. Der Rückstand wird chromatographiert (Acetonitril/Wasser (0,05 % Trifluoresigsäure 20/80 nach 100/0)). Man erhielt 120 mg Methyl-4-[{[2,6-dichlor-3-(5-chlor-2-thienyl)phenyl]acetyl}(2,2-difluorethyl)amino]-1,3-thiazol-5-carboxylat.
¹HNMR (400 MHz, CDCl₃): 8.88 (s, 1H), 7.31 (d, 1H), 7.28 (d, 1H), 7.01 (d, 1H), 6.90 (d, 1 H), 6.10 (tt, 1 H), 4.12 (td, 2H), 3.95 (s, 3H), 3.93 (s, 2H).

### 4.4. Herstellung von 6-[2,6-Dichlor-3-(5-chlor-2-thienyl)phenyl]-4-(2,2-difluorethyl)-7-hydroxy[1,3]thiazolo[4,5-b]pyridin-5(4H)-on (Verbindung I-a-14)

120 mg (0.23 mmol) Methyl-4-[{[2,6-dichlor-3-(5-chlor-2-thienyl)phenyl]acetyl}(2,2-difluorethyl)amino]-1,3-thiazol-5-carboxylat wurden in 10 ml THF gelöst und unter Argon-Atmosphäre mit 6.6 mg (0.27 mmol) Natriumhydrid versetzt. Die Reaktionsmischung wurde 4h bei RT gerührt. Danach wurde das THF unter Vakuum abgezogen und der Rückstand mit Wasser aufgenommen. Die wässrige Phase wurde mit Dichlormethan extrahiert, die organische Phase getrocknet und eingedampft, die wässrige Phase wurde mit 1 N Salzsäure angesäuert. Nach Extraktion mit Dichlormethan wurde die organische Phase getrocknet und eingedampft. Die Produkt enthaltenden Rückstände wurden chromatographiert (Acetonitril/Wasser (0,05 % Trifluoressigsäure) 20/80 nach 100/0)). Man erhielt 50 mg 6-[2,6-Dichlor-3-(5-chlor-2-thienyl)phenyl]-4-(2,2-difluorethyl)-7-hydroxy[1,3]thiazolo[4,5-b]pyridin-5(4H)-on.

In Analogie zu den oben genannten Herstellungmethoden erhält man die in nachfolgenden Tabellen genannten Verbindungen der Formel (I). Die Abkürzungen Me und Et stehen für Methyl und Ethyl.

**Tabelle 71: Erfindungsgemäße Verbindungen, worin R¹ für Wasserstoff steht, und die anderen Substituenten die in Tabelle 71 genannten Bedeutungen haben. R² bedeutet Methyl (a), 2,2-Difluorethyl (b), Propargyl (c) oder 2,2,2-Trifluorethyl (d). Ph bedeutet Phenyl.**

| 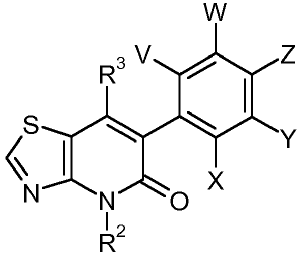 | | | | | | | | |
|---|---|---|---|---|---|---|---|---|
| Nr | R² | R³ | X | Y | Z | W | V | ¹H-NMR |
| I-a-1 | a | OH | Et | H | 2,4-Cl₂-Ph | H | Cl | |
| I-a-2 | b | OH | Et | H | 2,4-Cl₂-Ph | H | Cl | 400 MHz, CDCl₃: 8.97 (s, 1 H), 7.50 (dd, 2H), 7.33 (3H), 6.29 (tt, 1 H), 4.88 (m, 2H), 2.55 (m, 2H), 1.15 (t, 3H) |
| I-a-3 | c | OH | Et | H | 2,4-Cl₂-Ph | H | Cl | |
| I-a-4 | a | OH | Et | H | 5-Cl-Thien-2-yl | H | Cl | |
| I-a-5 | b | OH | Et | H | 5-Cl-Thien-2-yl | H | Cl | 400 MHz, DMSO-d₆: 11.43 (br s, 1 H), 9.41 (s, 1H), 7.63 (d, 1 H), 7.54 (d, 1 H), 7.48 (d, 1 H), 7.21 (d, 1 H), 6.34 (tt, 1 H), 4.75 (m, 2H), 2.42 (q, 2H), 1.04 (t, 3H) |
| I-a-6 | c | OH | Et | H | 5-Cl-Thien-2-yl | H | Cl | 400 MHz, CDCl₃: 9.00 (s, 1H), 7.55 (d, 1 H), 7.40 (d, 1H), 7.14 (d, 1 H), 6.93 (d, 1 H), 5.81 (br s, 1 H), 5.29 (dd, 1 H), 5.22 (dd, 1H), 2.54 (m, 2H), 2.22 (t, 1 H), 1.15 (t, 3H) |
| I-a-7 | a | OH | Cl | 4-Cl-Ph | H | H | Cl | 400 MHz, DMSO-d₆: 11.36 (br s, 1 H), 9.41 (s, 1 H), 7.61 (d, 1 H), 7.55 (m, 2H), 7.45 (m, 3H), 3.71 (s, 3H) |
| I-a-8 | b | OH | Cl | 4-Cl-Ph | H | H | Cl | 400 MHz, CDCl₃: 8.94 (s, 1H), 7.42 (d, 1H), 7.35 (m, 2H), 7.25 (m, 3H), 6.22 (tt, 1H), 4.83 (td, 2H) |
| I-a-9 | c | OH | Cl | 4-Cl-Ph | H | H | Cl | 400 MHz, DMSO-d₆: 11.60 (br s, 1H), 9.45 (s, 1H), 7.62 (d, 1H), 7.55 (m, 2H), 7.46 (m, 3H), 5.05 (d, 2H), 3.16 (t, 1H) |
| I-a-10 | a | OH | Cl | 2,4-Cl₂-Ph | H | H | Cl | |
| I-a-11 | b | OH | Cl | 2,4-Cl₂-Ph | H | H | Cl | 400 MHz, CDCl₃: 8.97 (s, 1H), 7.56 (d, 1H), 7.51 (m, 1H), 7.31 (m, 3H), 6.27 (tt, 1H), 5.41 (br s, 1H), 4.87 (m, 2H) |
| I-a-12 | c | OH | Cl | 2,4-Cl2-Ph | H | H | Cl | |
| I-a-13 | a | OH | Cl | 5-Cl-Thien-2-yl | H | H | Cl | |
| I-a-14 | b | OH | Cl | 5-Cl-Thien-2-yl | H | H | Cl | 400 MHz, CDCl₃: 8.98 (s, 1H), 7.52 (br s, 2H), 7.11 (d, 1H), 6.93 (d, 1H), 6.28 (tt, 1H), 4.87 (td, 2H) |
| I-a-15 | c | OH | Cl | 5-Cl-Thien-2-yl | H | H | Cl | |
| I-a-16 | a | OH | Cl | 4-Cl-Ph | H | H | F | 400 MHz, DMSO-d₆: 11.48 (br s, 1H), 9.41 (s, 1H), 7.54 (m, 2H), 7.47 (m, 3H), 7.36 (t, 1H), 3.71 (s, 3H) |
| I-a-17 | b | OH | Cl | 4-Cl-Ph | H | H | F | 400 MHz, DMSO-d₆: 11.81 (br s, 1H), 9.44 s, 1H), 7.54 (m, 2H), 7.47 (m, 3H), 7.37 (t, 1H), 6.36 (tt, 1H), 4.75 (td, 2H) |
| I-a-18 | c | OH | Cl | 4-Cl-Ph | H | H | F | 400 MHz, DMSO-d₆: 11.71 (br s, 1H), 9.45 (s, 1H), 7.55 (m, 2H), 7.48 (m, 3H), 7.37 (t, 1H), 5.05 (d, 2H), 3.17 (t, 1H) |
| I-a-19 | a | OH | Cl | 2,4-Cl₂-Ph | H | H | F | 400 MHz, CDCl₃: 8.94 (s, 1H), 7.49 (d, 1H), 7.30 (m, 2H), 7.19 (m, 2H), 3.90 (s, 3H) |
| I-a-20 | b | OH | Cl | 2,4-Cl₂-Ph | H | H | F | 400 MHz, DMSO-d₆: 9.43 (s, 1H), 7.77 (dd, 1H), 7.54 (dt, 1H), 7.40 (m, 3H), 6.36 (tq, 1H), 4.74 (m, 2H) |
| I-a-21 | c | OH | Cl | 2,4-Cl₂-Ph | H | H | F | 400 MHz, CDCl₃: 9.01 (s, 1H), 7.54 (m, 1H), 7.35 (m, 2H), 7.25 (m, 2H), 5.25 (d, 2H), 2.23 (t, 1H) |
| I-a-22 | a | OH | Cl | 5-Cl-Thien-2-yl | H | H | F | 400 MHz, DMSO-d₆: 11.51 (br s, 1H), 9.42 (s, 1H), 7.69 (dd, 1H), 3.66 (t, 1H), 7.27 (d, 1H), 7.21 (d, 1H), 3.71 (s, 3H) |
| I-a-23 | a | OC(O)OEt | Cl | 5-Cl-Thien-2-yl | H | H | F | 400 MHz, CDCl₃: 8.99 (s, 1H), 7.50 (dd, 1H), 7.14 (t, 1H), 7.04 (d, 1H), 6.91 (d, 1H), 4.23 (q, 2H), 3.97 (s, 3H), 1.24 (t, 3H) |
| I-a-24 | b | OH | Cl | 5-Cl-Thien-2-yl | H | H | F | 400 MHz, CDCl₃: 8.96 (s, 1H), 7.51 (dd, 1H), 7.14 (t, 1H), 7.02 (d, 1H), 6.91 (d, 1H), 6.25 (tt, 1H), 4.84 (td, 2H) |
| I-a-25 | c | OH | Cl | 5-Cl-Thien-2-yl | H | H | F | 400 MHz, CDCl₃: 9.00 (s, 1H), 7.54 (dd, 1H), 7.16 (t, 1H), 7.04 (d, 1H), 6.91 (d, 1H), 5.24 (m, 2H), 2.23 (t, 1H) |
| I-a-26 | c | OC(O)OEt | Cl | 5-Cl-Thien-2-yl | H | H | F | 400 MHz, CDCl₃: 9.04 (s, 1H), 7.50 (dd, 1H), 7.14 (t, 1H), 7.04 (d, 1H), 6.91 (d, 1H), 5.30 (s, 2H), 4.23 (q, 2H), 2.26 (t, 1H), 1.24 (t, 3H) |
| I-a-27 | a | OH | Et | H | 4-Cl-Ph | H | Cl | |
| I-a-28 | b | OH | Et | H | 4-Cl-Ph | H | Cl | 400 MHz, CDCl₃: 8.97 (s, 1H), 7.60 (d, 1H), 7.53 (m, 2H), 7.45 (m, 3H), 6.29 (tt, 1H), 5.80 (br s, 1H), 4.88 (m, 2H), 2.56 (m, 2H), 1.16 (t, 3H) |
| I-a-29 | c | OH | Et | H | 4-Cl-Ph | H | Cl | 400 MHz, CDCl₃: 9.00 (s, 1H), 7.59 (d, 1H), 7.53 (m, 2H), 7.45 (m, 3H), 5.84 (br s, 1H), 5.30 (dd, 1H), 5.22 (dd, 1H), 2.57 (m, 2H), 2.22 (t, 1H), 1.16 (t, 3H) |
| I-a-30 | a | OH | H | H | 4-Cl-Ph | H | Cl | 400 MHz, CDCl₃: 8.93 (s, 1H), 7.73 (d, 1H), 7.55 (dd, 1H), 7.51 (m, 2H), 7.44 (m, 2H), 6.11 (br s, 1H), 3.89 (s, 3H) |
| I-a-31 | b | OH | H | H | 4-Cl-Ph | H | Cl | 400 MHz, DMSO-d₆: 11.50 (br s, 1H), 9.42 (s, 1H), 7.83 (d, 1H), 7.79 (m, 2H), 7.68 (dd, 1H), 7.56 (m, 2H), 7.41 (d, 1H), 6.36 (tt, 1H), 4.75 (td, 2H) |
| I-a-32 | b | OCH₂-CHF₂ | H | H | 4-Cl-Ph | H | Cl | 400 MHz, CDCl₃: 8.97 (s, 1H), 7.59 (m, 2H), 7.55 (m, 1H), 7.52 (m, 1H), 7.50 (m, 1H), 7.41 (m, 2H), 6.30 (tt, 1H), 5.82 (tt, 1H), 4.88 (td, 2H), 3.92 (m, 2H) |
| I-a-33 | c | OH | H | H | 4-Cl-Ph | H | Cl | 400 MHz, CDCl₃: 9.00 (s, 1H), 7.75 (d, 1H), 7.58 (dd, 1H), 7.51 (m, 3H), 7.45 (m, 2H), 5.29 (dd, 1H), 2.60 (dd, 1H), 2.23 (t, 1H) |
| I-a-34 | a | OH | H | 4-Cl-Ph | H | H | Cl | 400 MHz, CDCl₃: 8.95 (s, 1H), 7.63 (m,1H), 7.57 (m, 2H), 7.49 (m, 2H), 7.39 (m, 2H), 5.95 (s, 1H), 3.91 (s, 3H) |
| I-a-35 | b | OH | H | 4-Cl-Ph | H | H | Cl | 400 MHz, CDCl₃: 8.96 (s, 1H), 7.64 (m, 1H), 7.58 (m, 2H), 7.49 (m, 2H), 7.40 (m, 2H), 6.29 (tt, 1H), 6.15 (br s, 1H), 4.86 (td, 2H) |
| I-a-36 | b | OC(O)SMe | H | 4-Cl-Ph | H | H | Cl | 400 MHz, CDCl₃: 8.99 (s, 1H), 7.57 (d, 1H), 7.53 (dd, 1H), 7.49 (m, 2H), 7.46 (m, 1H), 7.40 (m, 2H), 6.32 (tt, 1H), 4.92 (td, 2H), 2.28 (s, 3H) |
| I-a-37 | c | OH | H | 4-Cl-Ph | H | H | Cl | 400 MHz, DMSO-d₆: 11.40 (br s, 1H), 9.43 (s, 1H), 7.72 (m, 3H), 7.61 (m, 2H), 7.51 (m, 2H), 5.05 (d, 2H), 3.16 (t, 1H) |
| I-a-38 | a | OH | H | 4-Cl-Ph | H | H | Me | 400 MHz, CDCl₃: 8.93 (s, 1H), 7.53 (dd, 1H), 7.48 (m, 2H), 7.44 (m, 2H), 7.36 (m, 2H), 5.92 (br s, 1H), 3.91 (s, 3H), 2.25 (s, 3H) |
| I-a-39 | b | OH | H | 4-Cl-Ph | H | H | Me | 400 MHz, CDCl₃: 8.95 (s, 1H), 7.57 (dd, 1H), 7.51 (m, 2H), 7.46 (m, 2H), 7.39 (m, 2H), 6.30 (tt, 1H), 6.06 (br s, 1H), 4.88 (m, 2H), 2.26 (s, 3H) |
| I-a-40 | c | OH | H | 4-Cl-Ph | H | H | Me | 400 MHz, CDCl₃: 8.98 (s, 1H), 7.54 (m, 1H), 7.50 (m, 2H), 7.44 (m, 2H), 7.38 (m, 2H), 5.30 (s, 1H), 5.26 (dd, 2H), 2.26 (s, 3H), 2.23 (t, 3H) |
| I-a-41 | c | OC(O)SMe | H | 4-Cl-Ph | H | H | Me | 400 MHz, CDCl₃: 9.00 (s, 1H), 7.49 (m, 3H), 7.36 (m, 4H), 5.29 (d, 2H), 2.25 (t, 1H), 2.24 (s, 3H), 2.22 (s, 3H) |
| I-a-42 | c | OC(O)CMe₂ | H | 4-Cl-Ph | H | H | Me | 400 MHz, CDCl₃: 8.98 (s, 1H), 7.47 (m, 3H), 7.35 (m, 4H), 5.30 (d, 2H), 2.52 (spt, 1H), 2.26 (t, 1H), 2.23 (s, 3H), 0.92 (d, 3H), 0.90 (d, 3H) |
| I-a-43 | a | OC(O)OEt | Cl | 2,4-Cl₂-Ph | H | H | F | 400 MHz, CDCl₃: 8.98 (s, 1H), 7.51 (m, 1H), 7.30 (m, 3H), 7.18 (m, 1H), 4.20 (q, 2H), 3.97 (s, 3H), 1.23 (t, 3H) |
| I-a-44 | a | OC(O)SMe | Cl | 2,4-Cl₂-Ph | H | H | F | 400 MHz, CDCl₃: 8.98 (s, 1H), 7.51 (m, 1H), 7.30 (m, 3H), 7.19 (m, 1H), 3.97 (s, 3H), 2.32 (s, 3H) |
| I-a-45 | b | OH | Cl | 3-Cl-Ph | H | H | Cl | 400 MHz, DMSO-d₆: 9.43 (s, 1H), 7.60 - 7.30 (m, 6H), 6.34 (m, 1H), 4.75 (m, 2H) |
| I-a-46 | b | OH | Cl | Ph | H | H | Cl | 400 MHz, DMSO-dₛ: 9.42 (s, 1H), 7.60 (d, 1H), 7.43 (m, 6H), 6.34 (m, 1H), 4.75 (m, 2H) |
| I-a-47 | d | OH | Cl | Ph | H | H | Cl | 400 MHz, DMSO-d₆: 9.43 (s, 1H), 7.61 (d, 1H), 7.43 (m, 6H), 5.14 (q, 2H) |
| I-a-48 | a | OH | Cl | Ph | H | H | Cl | 400 MHz, DMSO-d₆: 9.40 (s, 1H), 7.59 (d, 1H), 7.43 (m, 6H), 3.71 (s, 3H) |
| I-a-49 | b | OH | CF₃ | 4,5-Dihydro-1,2-oxazol-3-yl | H | H | H | 400 MHz, DMSO-d₆: 11.48 (br s, 1H); 9.41 (s, 1H); 7.75 (t, 1H); 7.58 (d, 1H); 7.41 (d, 1H); 6.32 (tt, 1H); 4.73 (tt, 2H); 4.42 (t, 2H); 3.34 (t, 2H) |
| I-a-50 | b | OCH₂CHF₂ | CF₃ | 4,5-Dihydro-1,2-oxazol-3-yl | H | H | H | 400 MHz, DMSO-d₆: 9.52 (s, 1H); 7.80 (t, 1H); 7.66 (d, 1H); 7.53 (d, 1H); 6.36 (tt, 1H); 6.12 (tt, 1H); 4.79 (tt, 2H); 4.43 (t, 2H); 9.63 (m, 2H); 3.35 (t, 2H) |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigt z.B. die Verbindung Nr. I-a-19 bei einer Aufwandmenge von 1280 g/ha eine mindestens 80%-ige Wirkung gegen Alopecurus myosuroides und Cyperus serotinus und gleichzeitig keinerlei Schädigung in Reis und Weizen. Die Verbindung Nr. I-a-32 zeigt bei einer Aufwandmenge von 1280 g/ha eine mindestens 80%-ige Wirkung gegen Polygonum convolvulus und gleichzeitig keinerlei Schädigung in Mais und Raps. Die Verbindungen Nr. I-a-38 und I-a-39 zeigen bei einer Aufwandmenge von 1280 g/ha jeweils eine mindestens 80%-ige gegen Echinochloa crus galli und Veronica persica und gleichzeitig keinerlei Schädigung in Mais und Raps. Die Verbindung Nr. I-a-42 zeigt bei einer Aufwandmenge von 1280 g/ha eine mindestens 80%-ige Wirkung gegen Lolium multiflorum und Setaria viridis und gleichzeitig keinerlei Schädigung in Weizen.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen z.B. die Verbindungen Nr. I-a-39 und I-a-42 bei einer Aufwandmenge von 320 g/ha eine mindestens 80%-ige Wirkung gegen Setaria viridis und gleichzeitig keinerlei Schädigung in Mais und Raps. Die Verbindung Nr. I-a-38 zeigt bei einer Aufwandmenge von 320 g/ha eine mindestens 80%-ige Wirkung gegen Echinochloa crus galli und Lolium multiflorum und gleichzeitig keinerlei Schädigung in Mais und Reis. Die Verbindung Nr. I-a-42 zeigt bei einer Aufwandmenge von 80 g/ha eine mindestens 80%-ige Wirkung gegen Echinochloa crus galli und Setaria viridis und gleichzeitig keinerlei Schädigung in Weizen, Mais und Reis.

### 3. Insektizide Wirkung

### Beispiel A

### Phaedon -Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohl-blattscheiben (Brassica pekinensis) werden mit einer Wirkstoffzubereitung der gewünschten Konzentration gespritzt und nach dem Abtrocknen mit Larven des Meerrettichblattkäfers (Phaedon cochleariae) besetzt. Nach 7 Tagen wird die Wirkung in % bestimmt. Dabei bedeutet 100 %, dass alle Käferlarven abgetötet wurden; 0 % bedeutet, dass keine Käferlarven abgetötet wurden. In diesem Test zeigen z. B. die folgenden Verbindungen der Herstellungsbeispiele Wirkung von 100 % bei einer Aufwandmenge von 500 g/ha: I-a-29, I-a-40.

### Beispiel B

### Spodoptera frugiperda -Test (SPODFR Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblatt-scheiben (Zea mays) werden mit einer Wirkstoffzubereitung der gewünschten Konzentration gespritzt und nach dem Abtrocknen mit Raupen des Heerwurms (Spodoptera frugiperda) besetzt. Nach 7 Tagen wird die Wirkung in % bestimmt. Dabei bedeutet 100 %, dass alle Raupen abgetötet wurden; 0 % bedeutet, dass keine Raupe abgetötet wurde. In diesem Test zeigen z. B. die folgenden Verbindungen der Herstellungsbeispiele Wirkung von 85 % bei einer Aufwandmenge von 500 g/ha: I-a-38, I-a-39, I-a-40. In diesem Test zeigen z. B. die folgenden Verbindungen der Herstellungsbeispiele Wirkung von 100 % bei einer Aufwandmenge von 500 g/ha: I-a-38, I-a-39.

### Beispiel C

### Tetranychus - test, OP-resistent (TETRUR Spritzbehandlung)

| | | |
|---|---|---|
| Lösungsmittel: | 78,0 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblatt-scheiben (Phaseolus vulgaris), die von allen Stadien der Gemeinen Spinnmilbe (Tetranychus urticae) befallen sind, werden mit einer Wirkstoffzubereitung der gewünschten Konzentration gespritzt. Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

In diesem Test zeigen z. B. die folgenden Verbindungen der Herstellungsbeispiele eine Wirkung von 85 % bei einer Aufwandmenge von 500 g/ha: I-a-8, I-a-37, I-a-38, I-a-39.

In diesem Test zeigen z. B. die folgenden Verbindungen der Herstellungsbeispiele eine Wirkung von 90 % bei einer Aufwandmenge von 500 g/ha: I-a-8, I-a-37, I-a-39. In diesem Test zeigen z. B. die folgenden Verbindungen der Herstellungsbeispiele eine Wirkung von 100 % bei einer Aufwandmenge von 500 g/ha: I-a-8.

## Patentansprüche

1. Thiazolopyridinone der Formel (I) oder deren Salze 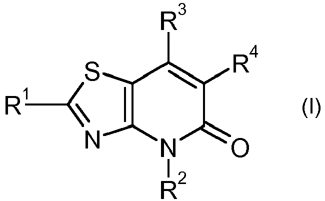 worin
R¹ bedeutet Wasserstoff, Halogen, Nitro, Cyano,
jeweils durch n Halogenatome substituiertes (C₁-C₆)-Alkyl, (C₂-C₆)-Alkenyl, (C₂-C₆)-Alkinyl, (C₁-C₆)-Alkoxy, (C₁-C₆)-Alkylthio, (C₁-C₆)-Alkylsulfinyl, (C₁-C₆)-Alkylsulfonyl, (C₃-C₆)-Cycloalkyl, (C₁-C₆)-Alkoxy-(C₁-C₆)-alkyl, Di-(C₁-C₆)-alkoxy-(C₁-C₆)-alkyl oder
(C₁-C₆)-Alkylthio-(C₁-C₆)-alkyl,
R² bedeutet Wasserstoff,
jeweils durch n Halogenatome substituiertes (C₁-C₆)-Alkyl, (C₂-C₆)-Alkenyl, (C₃-C₆)-Alkinyl, (C₃-C₆)-Cycloalkyl, (C₁-C₆)-Alkoxy-(C₁-C₆)-alkyl oder Di-(C₁-C₆)-alkoxy-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, (C₃-C₆)-Cycloalkyl-(C₁-C₆)-alkyl, Cyano-(C₁-C₆)-alkyl,
oder jeweils durch n Reste aus der Gruppe bestehend aus Halogen, (C₁-C₆)-Alkyl und (C₁-C₆)-Alkoxy substituiertes Phenyl oder Benzyl,
R³ bedeutet Hydroxy, O-C(=O)R⁷, O-C(=L)MR⁸, O-SO2R⁹, O-P(=L)R¹⁰R¹¹, O-C(=L)NR¹²R¹³, O-E oder O-R¹⁴,
R⁴ bedeutet R^{4a},
R^{4a} bedeutet durch n Reste R⁵ und einen Rest R⁶ substituiertes Aryl,
R⁵ bedeutet Wasserstoff, Halogen, Nitro, Cyano,
jeweils durch n Halogenatome substituiertes (C₁-C₆)-Alkyl, (C₂-C₆)-Alkenyl, (C₂-C₆)-Alkinyl, (C₁-C₆)-Alkoxy, (C₁-C₆)-Alkylthio, (C₁-C₆)-Alkylsulfinyl, (C₁-C₆)-Alkylsulfonyl, (C₃-C₆)-Cycloalkyl, (C₁-C₆)-Alkoxy-(C₁-C₆)-alkyl oder (C₁-C₆)-Alkylthio-(C₁-C₆)-alkyl,
R⁶ bedeutet jeweils durch s Reste R⁵ substituiertes Aryl oder Heteroaryl,
E bedeutet ein Metallionäquivalent oder ein Ammoniumion,
L bedeutet Sauerstoff oder Schwefel,
M bedeutet Sauerstoff oder Schwefel,
R⁷ bedeutet jeweils durch n Halogenatome substituiertes (C₁-C₆)-Alkyl, (C₂-C₆)-Alkenyl, (C₁-C₆)-Alkoxy-(C₁-C₆)-alkyl, Di-(C₁-C₆)-alkoxy-(C₁-C₆)-alkyl oder (C₁-C₆)-Alkylthio-(C₁-C₆)-alkyl,
einen durch n Reste aus der Gruppe bestehend aus Halogen, (C₁-C₆)-Alkyl und (C₁-C₆)-Alkoxy substituierten, vollständig gesättigten, 3- bis 6-gliedrigen Ring bestehend aus 3 bis 5 Kohlenstoffatomen und 1 bis 3 Heteroatomen aus der Gruppe bestehend aus Sauerstoff, Schwefel und Stickstoff,
oder jeweils durch n Reste aus der Gruppe bestehend aus Halogen, (C₁-C₆)-Alkyl und (C₁-C₆)-Alkoxy substituiertes (C₃-C₆)-Cycloalkyl, Phenyl, Phenyl-(C₁-C₆)-alkyl, Phenoxy-(C₁-C₆)-alkyl oder Heteroaryloxy-(C₁-C₆)-alkyl,
R⁸ bedeutet jeweils durch n Halogenatome substituiertes (C₁-C₆)-Alkyl, (C₂-C₆)-Alkenyl, (C₁-C₆)-Alkoxy-(C₁-C₆)-alkyl oder Di-(C₁-C₆)-alkoxy-(C₁-C₆)-alkyl,
oder jeweils durch n Reste aus der Gruppe bestehend aus Halogen, (C₁-C₆)-Alkyl und (C₁-C₆)-Alkoxy substituiertes (C₃-C₆)-Cycloalkyl, Phenyl oder Benzyl,
R⁹, R¹⁰, R¹¹ bedeuten unabhängig voneinander jeweils durch n Halogenatome substituiertes (C₁-C₆)-Alkyl, (C₁-C₆)-Alkoxy, N-(C₁-C₆)-Alkylamino, N,N-Di-(C₁-C₆)-Alkylamino, (C₁-C₆)-Alkylthio, (C₂-C₆)-Alkenyl oder (C₃-C₆)-Cycloalkylthio,
oder jeweils durch n Reste aus der Gruppe bestehend aus Halogen, (C₁-C₆)-Alkyl und (C₁-C₆)-Alkoxy substituiertes Phenyl, Benzyl, Phenoxy oder Phenylthio,
R¹² , R¹³ bedeuten unabhängig voneinander jeweils Wasserstoff,
durch n Halogenatome substituiertes (C₁-C₆)-Alkyl, (C₃-C₆)-Cycloalkyl, (C₂-C₆)-Alkenyl, (C₁-C₆)-Alkoxy oder (C₁-C₆)-Alkoxy-(C₁-C₆)-alkyl,
jeweils durch n Reste aus der Gruppe bestehend aus Halogen, (C₁-C₆)-Alkyl und (C₁-C₆)-Alkoxy substituiertes Phenyl oder Benzyl,
oder R¹² und R¹³ bilden gemeinsam mit dem N-Atom, an das sie gebunden sind,
einen 3- bis 6-gliedrigen Ring enthaltend 2 bis 5 Kohlenstoffatomen und jeweils 0 oder 1 Sauerstoff- oder Schwefelatome,
R¹⁴ bedeutet jeweils durch n Halogenatome substituiertes (C₁-C₆)-Alkyl, (C₂-C₆)-Alkenyl, (C₂-C₆)-Alkinyl, (C₁-C₆)-Alkoxy-(C₁-C₆)-alkyl, (C₁-C₆)-Alkylthio-(C₁-C₆)-alkyl oder Di-(C₁-C₆)-alkoxy-(C₁-C₆)-alkyl,
durch n Reste aus der Gruppe bestehend aus Halogen, (C₁-C₆)-Alkyl und (C₁-C₆)-Alkoxy substituiertes (C₃-C₆)-Cycloalkyl,
einen durch n Reste aus der Gruppe bestehend aus Halogen, (C₁-C₆)-Alkyl und (C₁-C₆)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
durch n Reste aus der Gruppe bestehend aus Halogen, (C₁-C₆)-Alkyl und (C₁-C₆)-Alkoxy substituiertes Phenyl, Phenyl-(C₁-C₆)-alkyl, Heteroaryl, Phenoxy-(C₁-C₆)-alkyl oder Heteroaryloxy-(C₁-C₆)-alkyl,
n bedeutet 0, 1, 2 oder 3,
s bedeutet 0, 1, 2 , 3 oder 4.

2. Thiazolopyridinone nach Anspruch 1, worin
R¹ bedeutet Wasserstoff,
jeweils durch n Halogenatome substituiertes (C₁-C₆)-Alkyl, (C₁-C₆)-Alkoxy, (C₁-C₆)-Alkylthio, (C₁-C₆)-Alkylsulfonyl, (C₃-C₆)-Cycloalkyl, (C₁-C₆)-Alkoxy-(C₁-C₆)-alkyl oder
(C1-C₆)-Al kylthio-(C₁-C₆)-alkyl,
R² bedeutet Wasserstoff,
jeweils durch n Halogenatome substituiertes (C₁-C₆)-Alkyl, (C₂-C₆)-Alkenyl, (C₃-C₆)-Alkinyl, (C₁-C₆)-Alkoxy-(C₁-C₆)-alkyl, (C₁-C₆)-Alkylthio-(C₁-C₆)-alkyl, (C₃-C₆)-Cycloalkyl-(C₁-C₆)-alkyl, Cyano-(C₁-C₆)-alkyl,
oder jeweils durch n Reste aus der Gruppe bestehend aus Halogen, (C₁-C₆)-Alkyl und (C₁-C₆)-Alkoxy substituiertes Phenyl oder Benzyl,
R³ bedeutet Hydroxy, O-C(=O)R⁷, O-C(=L)MR⁸, O-SO₂R⁹, O-P(=L)R¹⁰R¹¹, O-C(=L)NR¹²R¹³, O-E oder O-R¹⁴,
R⁴ bedeutet R^{4a},
R^{4a} bedeutet durch einen, zwei oder drei Reste R⁵ und einen Rest R⁶ substituiertes Phenyl,
R⁵ bedeutet Wasserstoff, Halogen,
jeweils durch n Halogenatome substituiertes (C₁-C₆)-Alkyl, (C₂-C₆)-Alkenyl, (C₂-C₆)-Alkinyl, (C₁-C₆)-Alkoxy, (C₃-C₆)-Cycloalkyl oder (C₁-C₆)-Alkoxy-(C₁-C₆)-alkyl,
R⁶ bedeutet durch n Reste R⁵ substituiertes Phenyl oder durch einen, zwei oder drei Reste R⁵ substituiertes Heteroaryl,
E bedeutet ein Metallionäquivalent oder ein Ammoniumion,
L bedeutet Sauerstoff oder Schwefel,
M bedeutet Sauerstoff oder Schwefel,
R⁷ bedeutet jeweils durch n Halogenatome substituiertes (C₁-C₆)-Alkyl, (C₂-C₆)-Alkenyl, (C₁-C₆)-Alkoxy-( C₁-C₆)-alkyl, Di-(C₁-C₆)-alkoxy-( C₁-C₆)-alkyl oder (C₁-C₆)-Alkylthio-(C₁-C₆)-alkyl,
einen durch n Reste aus der Gruppe bestehend aus Halogen, (C₁-C₆)-Alkyl und (C₁-C₆)-Alkoxy substituierten, vollständig gesättigten, 3- bis 6-gliedrigen Ring bestehend aus 3 bis 5 Kohlenstoffatomen und 1 bis 3 Heteroatomen aus der Gruppe bestehend aus Sauerstoff, Schwefel und Stickstoff,
oder jeweils durch n Reste aus der Gruppe bestehend aus Halogen, (C₁-C₆)-Alkyl und (C₁-C₆)-Alkoxy substituiertes (C₃-C₆)-Cycloalkyl, Phenyl, Phenyl-(C₁-C₆)-alkyl, Phenoxy-(C₁-C₆)-alkyl oder Heteroaryloxy-(C₁-C₆)-alkyl,
R⁸ bedeutet jeweils durch n Halogenatome substituiertes (C₁-C₆)-Alkyl, (C₂-C₆)-Alkenyl, (C₁-C₆)-Alkoxy-(C₁-C₆)-alkyl oder Di-(C₁-C₆)-alkoxy-(C₁-C₆)-alkyl,
oder jeweils durch n Reste aus der Gruppe bestehend aus Halogen, (C₁-C₆)-Alkyl und (C₁-C₆)-Alkoxy substituiertes (C₃-C₆)-Cycloalkyl, Phenyl oder Benzyl,
R⁹, R¹⁰, R¹¹ bedeuten unabhängig voneinander jeweils durch n Halogenatome substituiertes (C₁-C₆)-Alkyl, (C₁-C₆)-Alkoxy, N-(C₁-C₆)-Alkylamino, N,N-Di-(C₁-C₆)-Alkylamino, (C₁-C₆)-Alkylthio, (C₂-C₆)-Alkenyl oder (C₃-C₆)-Cycloalkylthio,
oder jeweils durch n Reste aus der Gruppe bestehend aus Halogen, (C₁-C₆)-Alkyl und (C₁-C₆)-Alkoxy substituiertes Phenyl, Benzyl, Phenoxy oder Phenylthio,
R¹², R¹³ bedeuten unabhängig voneinander jeweils Wasserstoff,
durch n Halogenatome substituiertes (C₁-C₆)-Alkyl, (C₃-C₆)-Cycloalkyl, (C₂-C₆)-Alkenyl, (C₁-C₆)-Alkoxy oder (C₁-C₆)-Alkoxy-(C₁-C₆)-alkyl,
jeweils durch n Reste aus der Gruppe bestehend aus Halogen, (C₁-C₆)-Alkyl und
(C₁-C₆)-Alkoxy substituiertes Phenyl oder Benzyl,
oder R¹² und R¹³ bilden gemeinsam mit dem N-Atom, an das sie gebunden sind,
einen 3- bis 6-gliedrigen Ring enthaltend 2 bis 5 Kohlenstoffatomen und jeweils 0 oder 1 Sauerstoff- oder Schwefelatome,
R¹⁴ bedeutet jeweils durch n Halogenatome substituiertes (C₁-C₆)-Alkyl, (C₂-C₆)-Alkenyl, (C₂-C₆)-Alkinyl, (C₁-C₆)-Alkoxy-(C₁-C₆)-alkyl, (C₁-C₆)-Alkylthio-(C₁-C₆)-alkyl oder Di-(C₁-C₆)-alkoxy-(C₁-C₆)-alkyl,
durch n Reste aus der Gruppe bestehend aus Halogen, (C₁-C₆)-Alkyl und (C₁-C₆)-Alkoxy substituiertes (C₃-C₆)-Cycloalkyl,
einen durch n Reste aus der Gruppe bestehend aus Halogen, (C₁-C₆)-Alkyl und (C₁-C₆)-Alkoxy substituierten, vollständig gesättigten, 3- bis 6-gliedrigen Ring bestehend aus 3 bis 5 Kohlenstoffatomen und 1 bis 3 Heteroatomen aus der Gruppe Sauerstoff, Schwefel und Stickstoff,
durch n Reste aus der Gruppe bestehend aus Halogen, (C₁-C₆)-Alkyl und (C₁-C₆)-Alkoxy substituiertes Phenyl, Phenyl-(C₁-C₆)-alkyl, Heteroaryl, Phenoxy-(C₁-C₆)-alkyl oder Heteroaryloxy-(C₁-C₆)-alkyl,
n bedeutet 0, 1, 2 oder 3,
s bedeutet 0, 1, 2 , 3 oder 4.

3. Herbizide Mittel, **gekennzeichnet durch** einen herbizid wirksamen Gehalt an mindestens einer Verbindung der Formel (I) gemäß Anspruch 1 oder 2.

4. Herbizide Mittel nach Anspruch 3 in Mischung mit Formulierungshilfsmitteln.

5. Herbizide Mittel nach Anspruch 3 oder 4 enthaltend mindestens einen weiteren pestizid wirksamen Stoff aus der Gruppe Insektizide, Akarizide, Herbizide, Fungizide, Safener und Wachstumsregulatoren.

6. Herbizide Mittel nach Anspruch 5 enthaltend einen Safener.

7. Herbizide Mittel nach Anspruch 6 enthaltend cyprosulfamid, cloquintocet-mexyl, mefenpyr-diethyl oder isoxadifen-ethyl.

8. Herbizide Mittel nach einem der Ansprüche 5 bis 7 enthaltend ein weiteres Herbizid.

9. Verfahren zur Bekämpfung unerwünschter Pflanzen, **dadurch gekennzeichnet, daß** man eine wirksame Menge mindestens einer Verbindung der Formel (I) gemäß Anspruch 1 oder 2 oder eines herbiziden Mittels nach einem der Ansprüche 3 bis 8 auf die Pflanzen oder auf den Ort des unerwünschten Pflanzenwachstums appliziert.

10. Verwendung von Verbindungen der Formel (I) gemäß Anspruch 1 oder 2 oder von herbiziden Mitteln nach einem der Ansprüche 3 bis 8 zur Bekämpfung unerwünschter Pflanzen.

11. Verwendung nach Anspruch 10, **dadurch gekennzeichnet, daß** die Verbindungen der Formel (I) zur Bekämpfung unerwünschter Pflanzen in Kulturen von Nutzpflanzen eingesetzt werden.

12. Verwendung nach Anspruch 11, **dadurch gekennzeichnet, daß** die Nutzpflanzen transgene Nutzpflanzen sind.

13. Insektizide Mittel, **gekennzeichnet durch** einen insektizid wirksamen Gehalt an mindestens einer Verbindung der Formel (I) gemäß Anspruch 1 oder 2.

14. Insektizide Mittel nach Anspruch 13 in Mischung mit Formulierungshilfsmitteln.

15. Insektizide Mittel nach Anspruch 13 oder 14 enthaltend mindestens einen weiteren pestizid wirksamen Stoff aus der Gruppe Insektizide, Akarizide, Herbizide, Fungizide, Safener und Wachstumsregulatoren.

16. Verfahren zur Bekämpfung unerwünschter Insekten, **dadurch gekennzeichnet, daß** man eine wirksame Menge mindestens einer Verbindung der Formel (I) gemäß Anspruch 1 oder 2 oder eines insektiziden Mittels nach einem der Ansprüche 13 bis 15 auf die Pflanzen oder auf die Insekten appliziert.

17. Verwendung von Verbindungen der Formel (I) gemäß Anspruch 1 oder 2 oder von insektiziden Mitteln nach einem der Ansprüche 13 bis 15 zur Bekämpfung unerwünschter Insekten.

## Claims

1. Thiazolopyridinones of the formula (I) or salts thereof 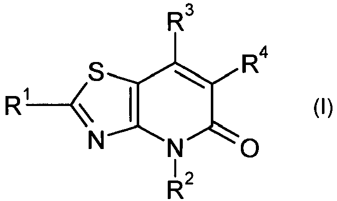 in which
R¹ represents hydrogen, halogen, nitro, cyano,
(C₁-C₆)-alkyl, (C₂-C₆)-alkenyl, (C₂-C₆)-alkynyl, (C₁-C₆)-alkoxy, (C₁-C₆)-alkylthio, (C₁-C₆)-alkylsulfinyl, (C₁-C₆)-alkylsulfonyl, (C₃-C₆)-cycloalkyl, (C₁-C₆)-alkoxy-(C₁-C₆)-alkyl, di-(_{C}1-_{C}6)-alkoxy-(C₁-C₆)-alkyl or (C₁-C₆)-alkylthio-(C₁-C₆)-alkyl, each of which is substituted by n halogen atoms,
R² represents hydrogen,
(C₁-C₆)-alkyl, (C₂-C₆)-alkenyl, (C₃-C₆)-alkynyl, (C₃-C₆)-cycloalkyl, (C₁-C₆)-alkoxy-(C₁-C₆)-alkyl or di-(C₁-C₆)-alkoxy-(C₁-C₆)-alkyl, (C₁-C₆)-alkylthio-(C₁-C₆)-alkyl, (C₁-C₆)-alkylsulfinyl-(C₁-C₆)-alkyl, (C₁-C₆)-alkylsulfonyl-(C₁-C₆)-alkyl, (C₃-C₆)-cycloalkyl-(C₁-C₆)-alkyl, cyano-(C₁-C₆)-alkyl, each of which is substituted by n halogen atoms,
or phenyl or benzyl, each of which is substituted by n radicals from the group consisting of halogen, (C₁-C₆)-alkyl and (C₁-C₆)-alkoxy,
R³ represents hydroxy, O-C(=O)R⁷, O-C(=L)MR⁸, O-SO₂R⁹, 0-P(=L)R¹⁰R¹¹, O-C(=L)NR¹²R¹³, O-E or O-R¹⁴,
R⁴ represents R^{4a},
R^{4a} represents aryl which is substituted by n radicals R⁵ and one radical R⁶,
R⁵ represents hydrogen, halogen, nitro, cyano,
(C₁-C₆)-alkyl, (C₂-C₆)-alkenyl, (C₂-C₆)-alkynyl, (C₁-C₆-alkoxy, (C₁-C₆)-alkylthio, (C₁-C₆)-alkylsulfinyl, (C₁-C₆)-alkylsulfonyl, (C₃-C₆) -cycloalkyl, (C₁-C₆)-alkoxy- (C₁-C₆)-alkyl or (C₁-C₆)-alkylthio-(C₁-C₆)-alkyl, each of which is substituted by n halogen atoms,
R⁶ represents aryl or heteroaryl, each of which is substituted by s radicals R⁵,
E represents a metal ion equivalent or an ammonium ion,
L represents oxygen or sulfur,
M represents oxygen or sulfur,
R⁷ represents (C₁-C₆)-alkyl, (C₂-C₆-alkenyl, (C₁-C₆)-alkoxy-(C₁-C₆)-alkyl, di-(C₁-C₆)-alkoxy-(C₁-C₆)-alkyl or (C₁-C₆)-alkylthio-(C₁-C₆)-alkyl, each of which is substituted by n halogen atoms,
a fully saturated 3- to 6-membered ring consisting of 3 to 5 carbon atoms and 1 to 3 heteroatoms from the group consisting of oxygen, sulfur and nitrogen, which ring is substituted by n radicals from the group consisting of halogen, (C₁-C₆)-alkyl and (C₁-C₆)-alkoxy,
or (C₃-C₆-cycloalkyl, phenyl, phenyl-(C₁-C₆)-alkyl, phenoxy-(C₁-C₆)-alkyl or heteroaryloxy-(C₁-C₆)-alkyl, each of which is substituted by n radicals from the group consisting of halogen, (C₁-C₆)-alkyl and (C₁-C₆)-alkoxy,
R⁸ represents (C₁-C₆)-alkyl, (C₂-C₆)-alkenyl, (C₁-C₆)-alkoxy-(C₁-C₆)-alkyl or di-(C₁-C₆)-alkoxy-(C₁-C₆)-alkyl, each of which is substituted by n halogen atoms,
or (C₃-C₆)-cycloalkyl, phenyl or benzyl, each of which is substituted by n radicals from the group consisting of halogen, (C₁-C₆)-alkyl and (C₁-C₆)-alkoxy,
R⁹, R¹⁰, R¹¹ independently of one another represent (C₁-C₆)-alkyl, (C₁-C₆)-alkoxy, N-(C₁-C₆)-alkylamino, N,N-di-(C₁-C₆)-alkylamino, (C₁-C₆)-alkylthio, (C₂-C₆)-alkenyl or (C₃-C₆)-cycloalkylthio, each of which is substituted by n halogen atoms, or phenyl, benzyl, phenoxy or phenylthio, each of which is substituted by n radicals from the group consisting of halogen, (C₁-C₆)-alkyl and (C₁-C₆)-alkoxy,
R¹², R¹³ independently of one another each represent hydrogen, (C₁-C₆)-alkyl, (C₃-C₆)-cycloalkyl, (C₂-C₆)-alkenyl, (C₁-C₆)-alkoxy or (C₁-C₆)-alkoxy-(C₁-C₆)-alkyl, substituted by n halogen atoms, phenyl or benzyl, each of which is substituted by n radicals from the group consisting of halogen, (C₁-C₆)-alkyl and (C₁-C₆)-alkoxy,
or R¹² and R¹³ together with the nitrogen atom to which they are attached form a 3- to 6-membered ring containing 2 to 5 carbon atoms and in each case 0 or 1 oxygen or sulfur atoms,
R¹⁴ represents (C₁-C₆)-alkyl, (C₂-C₆) -alkenyl, (C₂-C₆) -alkynyl, (C₁-C₆) -alkoxy- (C₁-C₆) -alkyl, (C₁-C₆) -alkylthio- (C₁-C₆) -alkyl or di-(C₁-C₆)-alkoxy-(C₁-C₆)-alkyl, each of which is substituted by n halogen atoms,
(C₃-C₆)-cycloalkyl substituted by n radicals from the group consisting of halogen, (C₁-C₆)-alkyl and (C₁-C₆)-alkoxy,
a fully saturated 3- to 6-membered ring consisting of 3 to 5 carbon atoms and 1 to 3 heteroatoms from the group consisting of oxygen, sulfur and nitrogen, which ring is substituted by n radicals from the group consisting of halogen, (C₁-C₆)-alkyl and (C₁-C₆)-alkoxy,
phenyl, phenyl-(C₁-C₆)-alkyl, heteroaryl, phenoxy-(C₁-C₆)-alkyl or heteroaryloxy-(C₁-C₆)-alkyl, substituted by n radicals from the group consisting of halogen, (C₁-C₆)-alkyl and (C₁-C₆)-alkoxy,
n represents 0, 1, 2 or 3,
s represents 0, 1, 2, 3 or 4.

2. Thiazolopyridinones according to Claim 1 in which
R¹ represents hydrogen,
(C₁-C₆)-alkyl, (C₁-C₆)-alkoxy, (C₁-C₆) -alkylthio, (C₁-C₆)-alkylsulfonyl, (C₃-C₆)-cycloalkyl, (C₁-C₆)-alkoxy-(C₁-C₆)-alkyl or (C₁-C₆)-alkylthio-(C₁-C₆)-alkyl, each of which is substituted by n halogen atoms,
R² represents hydrogen,
(C₁-C₆)-alkyl, (C₂-C₆)-alkenyl, (C₃-C₆)-alkynyl, (C₁-C₆)-alkoxy-(C₁-C₆)-alkyl, (C₁-C₆)-alkylthio-(C₁-C₆)-alkyl, (C₃-C₆)-cycloalkyl-(C₁-C₆)-alkyl, cyano-(C₁-C₆)-alkyl, each of which is substituted by n halogen atoms,
or phenyl or benzyl, each of which is substituted by n radicals from the group consisting of halogen, (C₁-C₆)-alkyl and (C₁-C₆)-alkoxy,
R³ represents hydroxy, O-C(=O)R⁷, O-C(=L)MR⁸, O-SO₂R⁹, OP(=L)R¹⁰R¹¹, O-C(=L)NR¹²R¹³, O-E or O-R¹⁴,
R⁴ represents R^{4a},
R^{4a} represents phenyl which is substituted by one, two or three radicals R⁵ and one radical R⁶,
R⁵ represents hydrogen, halogen,
(C₁-C₆)-alkyl, (C₂-C₆)-alkenyl, (C₂-C₆)-alkynyl, (C₁-C₆)-alkoxy, (C₃-C₆)-cycloalkyl or (C₁-C₆)-alkoxy-(C₁-C₆)-alkyl, each of which is substituted by n halogen atoms,
R⁶ represents phenyl substituted by n radicals R⁵ or heteroaryl substituted by one, two or three radicals R⁵,
E represents a metal ion equivalent or an ammonium ion,
L represents oxygen or sulfur,
M represents oxygen or sulfur,
R⁷ represents (C₁-C₆)-alkyl, (C₂-C₆)-alkenyl, (C₁-C₆)-alkoxy-(C₁-C₆)-alkyl, di-(C₁-C₆)-alkoxy-(C₁-C₆)-alkyl or (C₁-C₆)-alkylthio-(C₁-C₆)-alkyl, each of which is substituted by n halogen atoms,
a fully saturated 3- to 6-membered ring consisting of 3 to 5 carbon atoms and 1 to 3 heteroatoms from the group consisting of oxygen, sulfur and nitrogen, which ring is substituted by n radicals from the group consisting of halogen, (C₁-C₆)-alkyl and (C₁-C₆)-alkoxy,
or (C₃-C₆)-cycloalkyl, phenyl, phenyl-(C₁-C₆)-alkyl, phenoxy-(C₁-C₆)-alkyl or heteroaryloxy-(C₁-C₆)-alkyl, each of which is substituted by n radicals from the group consisting of halogen, (C₁-C₆)-alkyl and (C₁-C₆)-alkoxy,
R⁸ represents (C₁-C₆)-alkyl, (C₂-C₆)-alkenyl, (C₁-C₆)-alkoxy-(C₁-C₆)-alkyl or di-(C₁-C₆)-alkoxy-(C₁-C₆)-alkyl, each of which is substituted by n halogen atoms,
or (C₃-C₆)-cycloalkyl, phenyl or benzyl, each of which is substituted by n radicals from the group consisting of halogen, (C₁-C₆)-alkyl and (C₁-C₆)-alkoxy,
R⁹, R¹⁰, R¹¹ independently of one another represent (C₁-C₆)-alkyl, (C₁-C₆)-alkoxy, N-(C₁-C₆)-alkylamino, N,N-di-(C₁-C₆)-alkylamino, (C₁-C₆)-alkylthio, (C₂-C₆)-alkenyl or (C₃-C₆)-cycloalkylthio, each of which is substituted by n halogen atoms, or phenyl, benzyl, phenoxy or phenylthio, each of which is substituted by n radicals from the group consisting of halogen, (C₁-C₆)-alkyl and (C₁-C₆)-alkoxy,
R¹², R¹³ independently of one another each represent hydrogen, (C₁-C₆)-alkyl, (C₃-C₆) -cycloalkyl, (C₂-C₆) -alkenyl, (C₁-C₆) -alkoxy or (C₁-C₆)-alkoxy-(C₁-C₆)-alkyl, substituted by n halogen atoms, phenyl or benzyl, each of which is substituted by n radicals from the group consisting of halogen, (C₁-C₆)-alkyl and (C₁-C₆)-alkoxy,
or R¹² and R¹³ together with the nitrogen atom to which they are attached form a 3- to 6-membered ring containing 2 to 5 carbon atoms and in each case 0 or 1 oxygen or sulfur atoms,
R¹⁴ represents (C₁-C₆)-alkyl, (C₂-C₆) -alkenyl, (C₂-C₆) -alkynyl, (C₁-C₆) -alkoxy- (C₁-C₆) -alkyl, (C₁-C₆)-alkylthio- (C₁-C₆)-alkyl or di-(C₁-C₆)-alkoxy-(C₁-C₆)-alkyl, each of which is substituted by n halogen atoms,
(C₃-C₆)-cycloalkyl substituted by n radicals from the group consisting of halogen, (C₁-C₆)-alkyl and (C₁-C₆)-alkoxy,
a fully saturated 3- to 6-membered ring consisting of 3 to 5 carbon atoms and 1 to 3 heteroatoms from the group consisting of oxygen, sulfur and nitrogen, which ring is substituted by n radicals from the group consisting of halogen, (C₁-C₆)-alkyl and (C₁-C₆)-alkoxy,
phenyl, phenyl-(C₁-C₆)-alkyl, heteroaryl, phenoxy-(C₁-C₆)-alkyl or heteroaryloxy-(C₁-C₆)-alkyl, substituted by n radicals from the group consisting of halogen, (C₁-C₆)-alkyl and (C₁-C₆)-alkoxy,
n represents 0, 1, 2 or 3,
s represents 0, 1, 2, 3 or 4.

3. Herbicidal compositions, **characterized by** a herbicidally active content of at least one compound of the formula (I) according to Claim 1 or 2.

4. Herbicidal compositions according to Claim 3 in a mixture with formulation auxiliaries.

5. Herbicidal compositions according to Claim 3 or 4, comprising at least one further pesticidally active substance from the group of insecticides, acaricides, herbicides, fungicides, safeners and growth regulators.

6. Herbicidal compositions according to Claim 5, comprising a safener.

7. Herbicidal compositions according to Claim 6, comprising cyprosulfamide, cloquintocet-mexyl, mefenpyr-diethyl or isoxadifen-ethyl.

8. Herbicidal compositions according to any of Claims 5 to 7, comprising a further herbicide.

9. Method for controlling unwanted plants, **characterized in that** an effective amount of at least one compound of the formula (I) according to Claim 1 or 2 or of a herbicidal composition according to any of Claims 3 to 8 is applied to the plants or to the site of the unwanted plant growth.

10. Use of compounds of the formula (I) according to Claim 1 or 2 or of herbicidal compositions according to any of Claims 3 to 8 for controlling unwanted plants.

11. Use according to Claim 10, **characterized in that** the compounds of the formula (I) are used for controlling unwanted plants in crops of useful plants.

12. Use according to Claim 11, **characterized in that** the useful plants are transgenic useful plants.

13. Insecticidal compositions, **characterized in that** they comprise an insecticidally effective amount of at least one compound of the formula (I) according to Claim 1 or 2.

14. Insecticidal compositions according to Claim 13 in a mixture with formulation auxiliaries.

15. Insecticidal compositions according to Claim 13 or 14, comprising at least one further pesticidally active substance from the group of insecticides, acaricides, herbicides, fungicides, safeners and growth regulators.

16. Method for controlling unwanted insects, **characterized in that** an effective amount of at least one compound of the formula (I) according to Claim 1 or 2 or of an insecticidal composition according to any of Claims 13 to 15 is applied to the plants or to the insects.

17. Use of compounds of the formula (I) according to Claim 1 or 2 or of insecticidal compositions according to any of Claims 13 to 15 for controlling unwanted insects.

## Revendications

1. Thiazolopyridinones de formule (I) ou leurs
sels 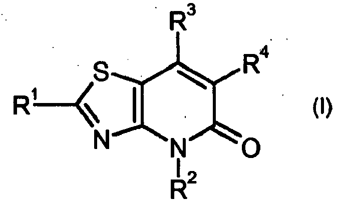 dans laquelle
R¹ signifie hydrogène, halogène, nitro, cyano ; (C₁-C₆)-alkyle, (C₂-C₆) -alcényle, (C₂-C₆) -alcynyle, (C₁-C₆)-alcoxy, (C₁-C₆) -alkylthio, (C₁-C₆)-alkylsulfinyle, (C₁-C₆)-alkylsulfonyle, (C₃-C₆)-cycloalkyle, (C₁-C₆)-alcoxy- (C₁-C₆)-alkyle, di-(C₁-C₆)-alcoxy-(C₁-C₆)-alkyle ou (C₁-C₆)-alkylthio-(C₁-C₆)-alkyle, à chaque fois substitué par n atomes d'halogène,
R² signifie hydrogène ; (C₁-C₆)-alkyle, (C₂-C₆)-alcényle, (C₃-C₆)-alcynyle, (C₃-C₆)-cycloalkyle, (C₁-C₆)-alcoxy-(C₁-C₆)-alkyle ou di-(C₁-C₆)-alcoxy-(C₁-C₆) -alkyle, (C₁-C₆) -alkylthio- (C₁-C₆) -alkyle, (C₁-C₆) -alkylsulfinyl- (C₁-C₆) -alkyle, (C₁-C₆)-alkylsulfonyl-(C₁-C₆) -alkyle, (C₃-C₆)-cycloalkyl-(C₁-C₆)-alkyle, cyano-(C₁-C₆)-alkyle, à chaque fois substitué par n atomes d'halogène ; ou phényle ou benzyle, à chaque fois substitué par n radicaux du groupe constitué par halogène, (C₁-C₆)-alkyle et (C₁-C₆)-alcoxy,
R³ signifie hydroxy, O-C(=O)R⁷, O-C(=L)MR⁸, O-SO₂R⁹, O-P(=L)R¹⁰R¹¹, O-C(=L)NR¹²R¹³, O-E ou O-R¹⁴,
R⁴ signifie R^{4a},
R^{4a} signifie aryle substitué par n radicaux R⁵ et par un radical R⁶,
R⁵ signifie hydrogène, halogène, nitro, cyano ; (C₁-C₆)-alkyle, (C₂-C₆)-alcényle, (C₂-C₆)-alcynyle, (C₁-C₆)-alcoxy, (C₁-C₆)-alkylthio, (C₁-C₆)-alkylsulfinyle, (C₁-C₆)-alkylsulfonyle, (C₃-C₆)-cycloalkyle, (C₁-C₆)-alcoxy-(C₁-C₆)-alkyle ou (C₁-C₆)-alkylthio-(C₁-C₆)-alkyle, à chaque fois substitué par n atomes d'halogène,
R⁶ signifie aryle ou hétéroaryle, à chaque fois substitué par s radicaux R⁵,
E signifie un équivalent d'ion métallique ou un ion d'ammonium,
L signifie oxygène ou azote,
M signifie oxygène ou azote,
R⁷ signifie (C₁-C₆)-alkyle, (C₂-C₆)-alcényle, (C₁-C₆)-alcoxy- (C₁-C₆) -alkyle, di-(C₁-C₆)-alcoxy-(C₁-C₆)-alkyle ou (C₁-C₆)-alkylthio-(C₁-C₆)-alkyle, à chaque fois substitué par n atomes d'halogène ; un cycle complètement saturé, de 3 à 6 chaînons, constitué par 3 à 5 atomes de carbone et par 1 à 3 hétéroatomes du groupe constitué par oxygène, soufre et azote, substitué par n radicaux du groupe constitué par halogène, (C₁-C₆)-alkyle et (C₁-C₆)-alcoxy ; ou (C₃-C₆)-cycloalkyle, phényle, phényl-(C₁-C₆)-alkyle, phénoxy-(C₁-C₆)-alkyle ou hétéroaryloxy-(C₁-C₆)-alkyle, à chaque fois substitué par n radicaux du groupe constitué par halogène, (C₁-C₆)-alkyle et (C₁-C₆)-alcoxy,
R⁸ signifie (C₁-C₆)-alkyle, (C₂-C₆)-alcényle, (C₁-C₆)-alcoxy-(C₁-C₆)-alkyle ou di-(C₁-C₆)-alcoxy-(C₁-C₆)-alkyle, à chaque fois substitué par n atomes d'halogène ; ou (C₃-C₆)-cycloalkyle, phényle ou benzyle, à chaque fois substitué par n radicaux du groupe constitué par halogène, (C₁-C₆)-alkyle et (C₁-C₆)-alcoxy,
R⁹, R¹⁰, R¹¹ signifient, indépendamment les uns des autres, (C₁-C₆)-alkyle, (C₁-C₆)-alcoxy, N-(C₁-C₆)-alkylamino, N,N-di-(C₁-C₆)-alkylamino, (C₁-C₆)-alkylthio, (C₂-C₆)-alcényle ou (C₁-C₆)-cycloalkylthio, à chaque fois substitué par n atomes d'halogène ; ou phényle, benzyle, phénoxy ou phénylthio, à chaque fois substitué par n radicaux du groupe constitué par halogène, (C₁-C₆)-alkyle et (C₁-C₆)-alcoxy,
R¹², R¹³ signifient, indépendamment l'un de l'autre, à chaque fois, hydrogène ; (C₁-C₆)-alkyle, (C₃-C₆)-cycloalkyle, (C₂-C₆)-alcényle, (C₁-C₆)-alcoxy ou (C₁-C₆)-alcoxy-(C₁-C₆)-alkyle, à chaque fois substitué par n atomes d'halogène ; phényle ou benzyle, à chaque fois substitué par n radicaux du groupe constitué par halogène, (C₁-C₆)-alkyle et (C₁-C₆)-alcoxy ; ou
R¹² et R¹³ forment, ensemble avec l'atome de N auquel ils sont liés, un cycle de 3 à 6 chaînons, contenant 2 à 5 atomes de carbone et à chaque fois 0 ou 1 atome d'oxygène ou de soufre,
R¹⁴ signifie (C₁-C₆)-alkyle, (C₂-C₆)-alcényle, (C₂-C₆)-alcynyle, (C₁-C₆)-alcoxy- (C₁-C₆)-alkyle, (C₁-C₆)-alkylthio-(C₁-C₆)-alkyle ou di-(C₁-C₆)-alcoxy-(C₁-C₆)-alkyle, à chaque fois substitué par n atomes d'halogène ; (C₃-C₆)-cycloalkyle substitué par n radicaux du groupe constitué par halogène, (C₁-C₆)-alkyle et (C₁-C₆)-alcoxy ; un cycle complètement saturé, de 3 à 6 chaînons, constitué par 3 à 5 atomes de carbone et 1 à 3 hétéroatomes du groupe formé par oxygène, soufre et azote, substitué par n radicaux du groupe constitué par halogène, (C₁-C₆)-alkyle et (C₁-C₆)-alcoxy; phényle, phényl-(C₁-C₆)-alkyle, hétéroaryle, phénoxy-(C₁-C₆)-alkyle ou étéroaryloxy-(C₁-C₆)-alkyle, substitués par n radicaux du groupe constitué par halogène, (C₁-C₆)-alkyle et (C₁-C₆)-alcoxy,
n vaut 0, 1, 2 ou 3,
s vaut 0, 1, 2, 3 ou 4.

2. Thiazolopyridinones selon la revendication 1, où
R¹ signifie hydrogène ; (C₁-C₆)-alkyle, (C₁-C₆)-alcoxy, (C₁-C₆)-alkylthio, (C₁-C₆)-alkylsulfonyle, (C₃-C₆)-cycloalkyle, (C₁-C₆)-alcoxy- (C₁-C₆)-alkyle ou (C₁-C₆)-alkylthio-(C₁-C₆)-alkyle, à chaque fois substitué par n atomes d'halogène,
R² signifie hydrogène ; (C₁-C₆)-alkyle, (C₂-C₆)-alcényle, (C₃-C₆)-alcynyle, (C₁-C₆)-alcoxy-(C₁-C₆)-alkyle, (C₁-C₆) -alkylthio-(C₁-C₆) -alkyle, (C₃-C₆)-cycloalkyl-(C₁-C₆)-alkyle, cyano-(C₁-C₆)-alkyle, à chaque fois substitué par n atomes d'halogène ; ou phényle ou benzyle, à chaque fois substitué par n radicaux du groupe constitué par halogène, (C₁-C₆)-alkyle et (C₁-C₆)-alcoxy,
R³ signifie hydroxy, O-C(=O)R⁷, O-C(=L)MR⁸, O-SO₂R⁹, O-P(=L)R¹⁰R¹¹, O-C(=L)NR¹²R¹³, O-E ou O-R¹⁴,
R⁴ signifie R^{4a},
R^{4a} signifie phényle substitué par un, deux ou trois radicaux R⁵ et par un radical R⁶,
R⁵ signifie hydrogène, halogène ; (C₁-C₆)-alkyle, (C₂-C₆)-alcényle, (C₂-C₆)-alcynyle, (C₁-C₆)-alcoxy, (C₃-C₆)-cycloalkyle ou (C₁-C₆)-alcoxy-(C₁-C₆)-alkyle, à chaque fois substitué par n atomes d'halogène,
R⁶ signifie phényle substitué par n radicaux R⁵ ou hétéroaryle substitué par un, deux ou trois radicaux R⁵,
E signifie un équivalent d'ion métallique ou un ion d'ammonium,
L signifie oxygène ou azote,
M signifie oxygène ou azote,
R⁷ signifie (C₁-C₆)-alkyle, (C₂-C₆)-alcényle, (C₁-C₆)-alcoxy-(C₁-C₆)-alkyle, di-(C₁-C₆)-alcoxy-(C₁-C₆)-alkyle ou (C₁-C₆)-alkylthio-(C₁-C₆)-alkyle, à chaque fois substitué par n atomes d'halogène ; un cycle complètement saturé, de 3 à 6 chaînons, constitué par 3 à 5 atomes de carbone et par 1 à 3 hétéroatomes du groupe constitué par oxygène, soufre et azote, substitué par n radicaux du groupe constitué par halogène, (C₁-C₆)-alkyle et (C₁-C₆)-alcoxy ; ou (C₃-C₆)-cycloalkyle, phényle, phényl-(C₁-C₆)-alkyle, phénoxy-(C₁-C₆)-alkyle ou hétéroaryloxy-(C₁-C₆)-alkyle, à chaque fois substitué par n radicaux du groupe constitué par halogène, (C₁-C₆)-alkyle et (C₁-C₆)-alcoxy,
R⁸ signifie (C₁-C₆)-alkyle, (C₁-C₆)-alcényle, (C₁-C₆)-alcoxy-(C₁-C₆)-alkyle ou di-(C₁-C₆)-alcoxy-(C₁-C₆)-alkyle, à chaque fois substitué par n atomes d'halogène ; ou (C₃-C₆)-cycloalkyle, phényle ou benzyle, à chaque fois substitué par n radicaux du groupe constitué par halogène, (C₁-C₆)-alkyle et (C₁-C₆)-alcoxy,
R⁹, R¹⁰, R¹¹ signifient, indépendamment les uns des autres, (C₁-C₆)-alkyle, (C₁-C₆)-alcoxy, N-(C₁-C₆)-alkylamino, N,N-di-(C₁-C₆)-alkylamino, (C₁-C₆)-alkylthio, (C₂-C₆)-alcényle ou (C₃-C₆)-cycloalkylthio, à chaque fois substitué par n atomes d'halogène ; ou phényle, benzyle, phénoxy ou phénylthio, à chaque fois substitué par n radicaux du groupe constitué par halogène, (C₁-C₆)-alkyle et (C₁-C₆)-alcoxy,
R¹², R¹³ signifient, indépendamment l'un de l'autre, à chaque fois, hydrogène ; (C₁-C₆)-alkyle, (C₃-C₆)-cycloalkyle, (C₂-C₆)-alcényle, (C₁-C₆)-alcoxy ou (C₁-C₆)-alcoxy-(C₁-C₆)-alkyle, à chaque fois substitué par n atomes d'halogène ; phényle ou benzyle, à chaque fois substitué par n radicaux du groupe constitué par halogène, (C₁-C₆)-alkyle et (C₁-C₆)-alcoxy ; ou
R¹² et R¹³ forment, ensemble avec l'atome de N auquel ils sont liés, un cycle de 3 à 6 chaînons, contenant 2 à 5 atomes de carbone et à chaque fois 0 ou 1 atome d'oxygène ou de soufre,
R¹⁴ signifie (C₁-C₆)-alkyle, (C₂-C₆)-alcényle, (C₂-C₆)-alcynyle, (C₁-C₆) -alcoxy- (C₁-C₆) -alkyle, (C₁-C₆)-alkylthio-(C₁-C₆)-alkyle ou di-(C₁-C₆)-alcoxy-(C₁-C₆)-alkyle, à chaque fois substitué par n atomes d'halogène ; (C₃-C₆)-cycloalkyle substitué par n radicaux du groupe constitué par halogène, (C₁-C₆)-alkyle et (C₁-C₆)-alcoxy ; un cycle complètement saturé, de 3 à 6 chaînons, constitué par 3 à 5 atomes de carbone et 1 à 3 hétéroatomes du groupe formé par oxygène, soufre et azote, substitué par n radicaux du groupe constitué par halogène, (C₁-C₆-alkyle et (C₁-C₆)-alcoxy ; phényle, phényl-(C₁-C₆)-alkyle, hétéroaryle, phénoxy-(C₁-C₆)-alkyle ou hétéroaryloxy-(C₁-C₆)-alkyle, substitués par n radicaux du groupe constitué par halogène, (C₁-C₆)-alkyle et (C₁-C₆)-alcoxy,
n vaut 0, 1, 2 ou 3,
s vaut 0, 1, 2, 3 ou 4,

3. Agents herbicides, **caractérisés par** une teneur active en tant qu'herbicide en au moins un composé de formule (I) selon la revendication 1 ou 2.

4. Agents herbicides selon la revendication 3 en mélange avec des adjuvants de formulation.

5. Agents herbicides selon la revendication 3 ou 4 contenant au moins une autre substance active en tant que pesticide du groupe formé par les insecticides, les acaricides, les herbicides, les fongicides, les phytoprotecteurs et les régulateurs de croissance.

6. Agents herbicides selon la revendication 5 contenant un phytoprotecteur.

7. Agents herbicides selon la revendication 6, contenant du cyprosulfamide, du cloquintocet-mexyl, du méfenpyr-diéthyl ou de l'isoxadifen-éthyl.

8. Agents herbicides selon l'une quelconque des revendications 5 à 7 contenant un autre herbicide.

9. Procédé pour lutter contre des plantes non souhaitées, **caractérisé en ce qu'**on applique une quantité active d'au moins un composé de formule (I) selon la revendication 1 ou 2 ou d'un agent herbicide selon l'une quelconque des revendications 3 à 8 sur les plantes ou sur le lieu de la croissance non souhaitée des plantes.

10. Utilisation de composés de formule (I) selon la revendication 1 ou 2 ou d'agents herbicides selon l'une quelconque des revendications 3 à 8 pour lutter contre des plantes non souhaitées.

11. Utilisation selon la revendication 10, **caractérisée en ce que** les composés de formule (I) sont utilisés pour lutter contre des plantes non souhaitées dans les cultures de plantes utiles.

12. Utilisation selon la revendication 11, **caractérisée en ce que** les plantes utiles sont des plantes utiles transgéniques.

13. Agents insecticides, **caractérisés par** une teneur active en tant qu'insecticide en au moins un composé de formule (I) selon la revendication 1 ou 2.

14. Agents insecticides selon la revendication 13 en mélange avec des adjuvants de formulation.

15. Agents insecticides selon la revendication 13 ou 14 contenant au moins une autre substance active en tant que pesticide du groupe formé par les insecticides, les acaricides, les herbicides, les fongicides, les phytoprotecteurs et les régulateurs de croissance.

16. Procédé pour lutter contre des insectes non souhaités, **caractérisé en ce qu'**on applique une quantité active d'au moins un composé de formule (I) selon la revendication 1 ou 2 ou d'un agent insecticide selon l'une quelconque des revendications 13 à 15 sur les plantes ou sur les insectes.

17. Utilisation de composés de formule (I) selon la revendication 1 ou 2 ou d'agents insecticides selon l'une quelconque des revendications 13 à 15 pour lutter contre des insectes non souhaités.
